# EUROPEAN PATENT SPECIFICATION

(11) **EP 2 285 337 B1**
(45) Date of publication and mention of the grant of the patent: **30.07.2014**
(21) Application number: 09757633.4
(22) Date of filing: 08.06.2009
(51) Int. Cl.: A61J 3/00, B01L 1/00, B65B 3/00, B65B 55/00, G07F 17/00, B65B 9/00

(54) **AUTOMATED WORKSTATION FOR THE SECURE PREPARATION OF A FINAL PRODUCT FOR MEDICAL OR PHARMACEUTICAL USE**
AUTOMATISCHE ARBEITSSTATION ZUR SICHEREN HERSTELLUNG EINES ENDPRODUKTS FÜR MEDIZINISCHE ODER PHARMAZEUTISCHE ANWENDUNGEN
POSTE DE TRAVAIL AUTOMATISE POUR LA PREPARATION SECURISEE D'UN PRODUIT FINI A USAGE MEDICAL OU PHARMACEUTIQUE

(30) Priority: 06.06.2008 FR 0803160
(43) Date of publication of application: 23.02.2011
(73) Proprietor: Pharmed S.A.M., 98000 Monaco (MC)
(72) Inventor: OSBORNE, Joel, Port Orange, FL 32128 (US)
(74) Representative: Gevers France
(86) International application number: PCT/EP2009/057034
(87) International publication number: WO 2009/147252

(56) References cited:
- EP-A- 1 433 705
- WO-A-95/15142
- WO-A-2005/096776
- WO-A-2006/124211
- US-B1- 7 146 781

## Description

The present invention relates to an automated workstation for the secure preparation of a final product for medical or pharmaceutical use of a determined composition and/or in determined quantities.

Such a final product can in particular be used to treat an identified patient. The final product is packaged in a final container such as a bag for intravenous IV use (IV bag) or a syringe, for example, of the single-use type, this type of final container being perfectly well known from the state of the art in the medical field.

Many products for medical use are injected into a patient from an intravenous bag, or "IV bag", into which a quantity of the product or medicine or drug has been introduced. Sometimes, the final product can be a mixture of different substances and/or diluents (dilutants).

This type of product for medical use can also be injected into the patient by means of a syringe.

The final product results from a preparation operation starting from different substances contained in vessels such as vials and/or flexible bags.

In a pharmacy, for example in a hospital, a pharmacist or a preparer mixes, in a preparation unit, a certain quantity of active substances with different volumes or different quantities of diluent(s) according to a medical prescription, which can in particular be intended for the treatment of an identified patient whose protocol requires different injections of determined dosage(s).

The present invention relates to an automated workstation for preparing and supplying such final products which ensures the greatest possible safety for the patients, in particular regarding the guarantee of the composition of the final product and the dosage, and for the pharmacy operators who must be protected against contact with hazardous substances.

The workstation is particularly suited to the preparation of cytotoxic medicines or drugs required in chemotherapy treatments for cancers. It is in fact well known that the active substances and principles used have the particular feature of exposing the operators who are regularly required to prepare and handle them to risks of cancers.

Because of this, there are many norms and standards relating to the correct and secure handling of these substances and drugs by hospital personnel and anticancer centres.

### SUMMARY OF THE INVENTION

To this end, the invention proposes an automated workstation, of the type disclosed in WO-A-2006/12411, for the preparation, starting from different substances, in powder and/or liquid form, each of which is contained in a vessel such as a vial or a bag, of a final product for medical or pharmaceutical use of a determined composition and/or in determined quantities, the final product being packaged in a container such as an IV bag for intravenous IV use or a syringe, the workstation comprising at least:
- an isolated chamber which delimits different internal functional areas;
- a storage area, for storing the filled vessels and containers;
- a transfer area ;
- a preparation area, for preparing the final products, which communicates at least with said storage area via said transfer area, and which comprises means for rapidly decontaminating the preparation area and its contents, said preparation area being isolated from the outside;
- a lock device for unloading the final products from the workstation ; and at least one robot or one programmable logic controller for handling the objects contained in the storage area and/or in the preparation area, characterized in that the workstation further comprises;
- a loading area (18), in particular for the vessels and containers, comprising means for rapidly decontaminating the loading area and its contents, said loading area being isolated from the outside and communicating with the storage area;
- a lock device for introducing the vessels and containers into the loading area (18).

According to other features of the inventive workstation:
- the workstation comprises at least two robots (R1, R2), or programmable logic controllers for handling objects, one of which is arranged in the storage area and the other one of which is arranged in the preparation area ;
- said loading area also communicates with said preparation area ;
- the workstation comprises labelling means, arranged in the storage area, which comprise a printer, in particular for printing labels in situ designed to be affixed to the vessels and/or containers ;
- the workstation comprises refrigerated storage means arranged in the storage area ;
- the workstation comprises means for packaging the final products in a protective envelope, these packaging means being arranged in the final product preparation area ;
- the workstation is housed in a flexible protective and isolating tent ;
- for the preparation of the products, the workstation contains closed needle-less transfer systems each of which mechanically prevents environmental pollutants from being transferred into the said system and hazardous substances or vapour concentrations from escaping towards the outside of the said system ;
- the workstation comprises analysis, checking and validation means which make it possible for a final product, after it has been prepared and before it leaves the automated station, to verify the conformity of the said final product ;
- the checking and validation of the product is performed without taking a sample directly from the final container, using the Raman spectrometry technology ;
- said verification includes a weight verification ;
- two of said areas are separated by a rotating vertical carrousel forming a rotating door with multiple shelving units which making it possible to transfer different elements and components from one area to the other area ;
- the carrousel forming a rotating door has two opposing faces, each of the said opposite faces being fitted, with a preparation wheel equipped with preparation means ;
- the introduction lock device is allows for the bio-decontamination of all the external or outside surfaces of all the elements and components that are placed inside the lock device by spraying suitable decontamination products.

Other features and benefits of the invention will become apparent from reading the following detailed description of the invention with reference to the drawings comprising the following figures.

### BRIEF DESCRIPTION OF CERTAIN FIGURES

Figure 1 is a diagrammatic plan view representing a first exemplary design and general layout of an automated workstation according to the invention.
Figures 2 to 4 are schematic representations similar to that of Figure 1 which represent some variants of design and general layout of an automated workstation according to the invention.
Figure 5 is a perspective view of an embodiment of an automated station covered with a protective and isolating tent.
Figure 6 is a view, from the same angle as Figure 5, illustrating the workstation 10 without the flexible protective and isolating tent.
Figure 7 is a plan view of the station of Figure 6.
Figure 8 is a left side view of the station of Figure 6.
Figure 9 is a bottom view of the station of Figure 6.
Figure 10 is a perspective view, from another viewing angle, of the station of Figure 6.
Figure 11 is a diagrammatic view similar to that of Figure 6 in which the roof or ceiling element of the station, and all its side walls, are represented in exploded view relative to the other parts of the workstation.
Figure 12 is a perspective view similar to that of Figure 10 which represents the station without its roof.
Figure 13 is a view similar to that of Figure 6 which represents the workstation without its roof element.
Figure 14 is a plan view of the station without its roof illustrated in Figures 12 and 13.
Figure 15 is a view similar to that of Figure 10 which represents the workstation without its roof element and without its side walls represented as an exploded view in Figure 11.
Figure 16 is a perspective view, from another viewing angle, of the workstation illustrated in Figure 15.
Figures 17 and 18 are two perspective views from two different angles which illustrate the main bottom parts forming the general structure of the workstation with, in addition, the loading mat and the loading lock device with double doors.

### ARCHITECTURE AND GENERAL DESCRIPTION OF THE AUTOMATED WORKSTATION

The workstation can be used as a safety device in as much as it protects the external operators from any exposures to hazardous elements, and in particular to cytotoxic drugs.

All of the various phases and steps of the method for preparing the final products take place in a medium contained in an ISO class 5, or higher, level "pharmaceutical" isolation environment, with cleaning and bio-decontamination means for maintaining a clean and sterile working environment around the workstation.

In particular, the workstation conforms to the ISO 14644 (clean room standard), ISO 14698 (bio-decontamination standard), and pharmaceutical GMP standards for the fabrication of sterile products and to the GAMP directives for automated aseptic filling software.

The workstation according to the invention comprises a chamber, isolated from the outside, which delimits different internal functional areas.

The chamber preferably functions permanently under a positive pressure with a general circulation of air that is one-way vertical, that is, from top to bottom, for example with a speed of about 0.3 to 0.6 m/s and without any removal or output to the external environment that is not equipped with appropriate filtering means. A negative pressure is also possible via a simple control switch.

The air used inside the chamber is filtered through HEPA or ULPA filters, and a system of secondary filters comprising HEPA filters and/or carbon filters that are used for the recirculation air and the neutralization of the active toxic elements. The addition of carbon filters reduces the contamination/recirculation of chemical products in gaseous form.

The workstation is equipped with means (not represented herein below) for continually controlling and monitoring the environment, inside and outside, such as particle counters, "living" particle counters ("viable particle counters"), pressure sensors, moisture sensors, differential pressure sensors, temperature sensors, air flow meters, etc.

All the control and monitoring data are stored and archived.

Provision is made to be able to use removable sealtight glove(s) entry systems (type F or DPTE). When such systems are used, in particular to minimize the requirements to "open" the isolated chamber, the handling robots or programmable logic controllers inside the workstation are stopped. To this end, usage or working sensors for these systems are provided.

The structure or frame of the station is sufficiently rigid to bear or support two robots which can be fixed in the top part, for example to the ceiling wall of the chamber. The storage and preparation areas must be "washable", that is, must be able to be washed with clean water with the possible addition of aseptic and detergent products.

If necessary, the various side walls which delimit the isolated chamber can be provided with windows to access different elements and equipment units within the workstation, and in particular in the storage and preparation areas.

Such windows must be airtight and provided with means for detecting their opening to automatically stop all moving devices inside, such as the handling robots.

So as to be able to implement decontamination means (means for cleaning by decontamination agents), the storage and preparation areas, and all of their contents, are compatible with gaseous rapid decontamination products such as hydrogen peroxide or chlorine peroxide.

The introduction lock device is preferably a double-door system that allows only one door to be opened at the same time. The introduction lock device allows for the bio-decontamination of all the external or outside surfaces of all the elements and components that are placed inside the lock device, for example by spraying suitable products.

The bio-decontamination gas is stored in an autonomous unit fully integrated into the workstation to allow in particular the gassing of the introduction lock device and of the storage and preparation areas by means of a system of tubes incorporated in the workstation with spraying nozzles distributed for that purpose, along the tubes, in the different areas concerned.

Inside the storage and preparation areas, a cleaning system is used, after one or more working sessions, to clean all the surfaces and all the contents. Such a system can, for example, be implemented by the robots. It is designed in such a way as to recover the cleaning and soiled products. It can, for example, be a continuous towel(ette) system, in the manner of a hand towel device in public places, which unwinds from an initial housing in which it is wound and kept moist and which, after use, is once again wound into a final housing to constitute a dry used and polluted towel(ette) roll, which can then be recovered.

If the workstation is not used in an ISO class 5 or higher clean room, the workstation is equipped with a "flexible clean room system" such as a tent, to safeguard the phases of loading, installing diluents in the peristaltic pump and the output of the final products. This closed volume covers all the workstation and it is also provided with particles, pressure, temperature and other sensors.

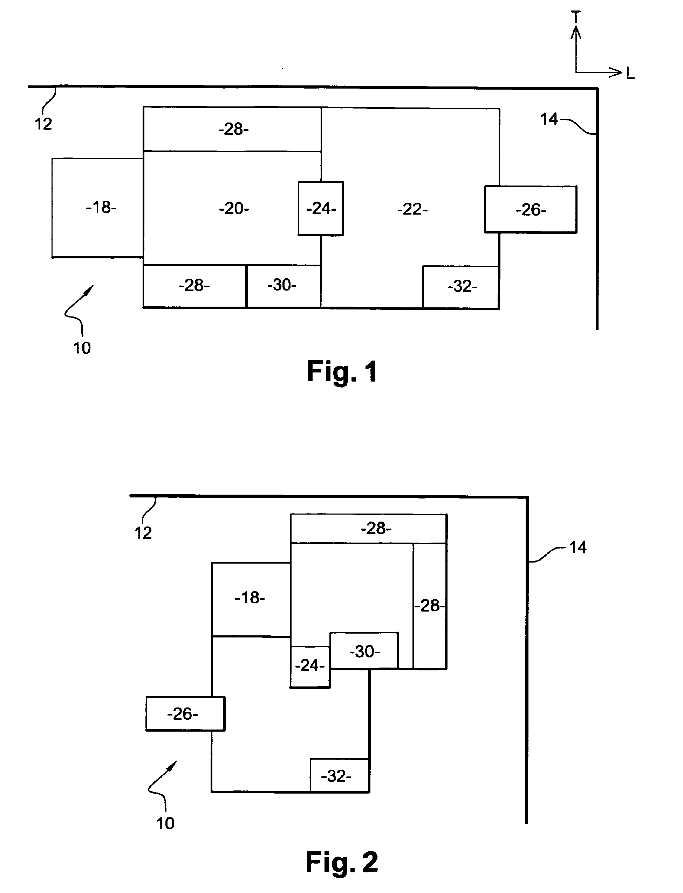

Figure 1 represents an automated workstation 10 which is in this case laid out, for example in a pharmacy room of a hospital, in a room delimited by at least two walls 12 and 14 at right angles.

The workstation 10 is essentially composed of an isolated chamber 16 which is delimited laterally by different vertically oriented walls which delimit in particular a loading area 18, a storage area 20, and a preparation area 22. The workstation 10 can move or be moved, for example by means of casters that are not represented, and it can thus be backed up to the walls 12 and 14.

As will be explained hereinbelow, the loading area 18 is designed to form a lock device, for the introduction of the elements and components present in the loading area for their introduction into the storage area 20, which communicates with the loading area 18.

The preparation area 22 communicates with the storage area 20 through a transfer area 24.

The station 10 also comprises means 26 arranged in the form of a lock device for unloading, or extracting, the products from the preparation area 22 towards the outside of the workstation 10.

As illustrated diagrammatically in Figure 1, the storage area 20 comprises groups 28 of shelves, some of which can belong to a refrigerated area forming refrigerated storage means arranged in the storage area.

In order to facilitate the circulation of air generally, in particular of air with the addition of decontamination gas and/or of refrigerated air in the refrigerated area for storing the products, the shelves (or shelving units) or equivalent means are preferably pierced.

In order to ensure a perfect circulation of air and/or of the decontamination gases, besides the fact that the shelving units and equivalent means are pierced, it is possible to provide, between the vertical shelving units supports or channels and the adjacent walls or partitions, air circulation spaces for the vertical passage from top to bottom and the lateral passage through the shelving units.

Concerning the general organization of the circulation of the air inside the isolated chamber of the workstation, an example that is particularly suited to the case where the robot is fixed or adapted to the ceiling of the chamber is the one in which the HEPA filters arranged in the top part are then used above any critical area, particularly in which a liquid transfer takes place.

The storage area 20 is also provided with a printer 30, in particular for printing in situ labels for the different elements and components contained in the workstation 10.

In this example, the preparation area 20 comprises an area 32 for securely treating and disposing of the waste issued from the final product preparation method as a whole.

As non-limiting examples, other design and layout arrangements are illustrated in Figures 2 to 4 in which identical numerical references are used to designate the same elements.

As a variant, in Figure 2, the loading area 18 communicates in particular both with the storage area 20 and with the preparation area 22.

Other layouts and designs are possible in the context of the present invention, depending on the planned uses and/or the internal arrangements of the workstation, such as the layout of the handling robots or programmable logic controllers and the possible circulation of these robots between the storage and preparation areas (as it is possible in Figure 1).

In a modular design, the dimensions of the storage area can be modified, and it can in particular be elongated according to storage capacity requirements.

The loading area 18, with its lock device function for introduction into the storage area 20, is used to introduce all the elements, components and consumables (disposables) into the storage area 20 and/or, when the layout permits, directly into the preparation area 22.

The storage area 20 is in particular the one containing all the vessels, such as vials, containing for example the cytotoxic products that are stored therein with the other consumable elements or components - that is, all the components or elements other than the containing vessels and the substances - which are required for the operation of the workstation, for the preparation of the final products, such consumables being, for example, labels and/or ribbons for the printer.

Of course, the storage area 20 also stores syringes of different sizes, needle-less adaptors or pins for vials, flexible bags, in particular of the intravenous IV type, universal supports for handling the vials and bags, cleaning equipments, gloves and envelopes (jackets) for the robots, absorbent mats for the shelving units, connecting pins, etc.

It is in the storage area 20 that a part of the preparation method is carried out, and in particular the final identification of the vials, the labelling of the containers for the prepared final products, the assembly of the adaptors on the vials, the visual recognition of certain elements, the decontamination by gaseous means, or bio-decontamination, of the external surfaces of everything contained in the storage area, weighing, etc.

The transfer area 24 is the area through which various elements or components pass from the storage area 20 to the preparation area 22.

The preparation area 22 is the one in which the final product preparation phase is implemented, in particular by mixing liquid products inside the vials, by weighing them, by transferring the liquid from a vial to a final container such as an intravenous flexible IV bag or a syringe. It is also where adding diluents and decontaminating is carried out.

The steps of validating the chemical composition and the concentrations of the prepared final products, before they leave the workstation, can also be carried out in this area 22.

The prepared final products are removed to the outside from the preparation area 22.

The preparation area 22 is also where all the waste is treated, in the part 32, with a view to their safe removal from the station 10.

The four main areas or volumes 18, 20, 22 and 24 constitute a global volume isolated from the external environment according to the class 5 to 7 ISO standards, the quality of which is verified by particles counters.

The loading area 18 with the entry lock device, in addition to its proper loading function, is also designed to constitute a rapid or "quick" decontamination area, in particular using gaseous means. This area is thus used for cleaning/decontamination.

The general design of the loading area 18 must be as simple as possible in order to reduce the duration of the operations and the risks associated with automation.

The benefit of implementing the decontamination inside the loading area 18 is the low volume of the latter which makes it possible to reduce the duration of the decontamination operations. This rapid decontamination can, of course, be complemented, regularly or otherwise, by the implementation of longer decontamination phases in the other areas and in particular in the storage area 20 and/or the preparation area 22.

When the volumes or areas to be decontaminated are greater, then the durations are longer, but these phases of decontamination of the complete areas or volumes 20 and 22 can, for example, be implemented outside the most intense periods of use of the workstation 10.

Loading is a phase that is critical to the whole of the process which, in particular, influences the overall rapidity and capacity.

It is important for the loading not to be any longer than when it is performed manually. Because of the nature of the decontamination gas whereby the decontamination phase takes at least 15 minutes for a limited volume, the design according to the invention comprising a loading and rapid decontamination area makes it possible to use a decontamination gas without affecting the working speed of the automated workstation.

Inside the loading area 18 forming an introduction and decontamination lock device with double doors, the decontamination cycle lasts approximately 15 minutes. It consists in increasing the temperature to facilitate the condensation of steam on the surface, in injecting the decontamination gas, and then in allowing an aeration phase to remove all the remaining traces of gas.

To this end, the following are needed: a pressurized decontaminant gas generator, an HEPA filter system, a carbon filter system (which is shortcircuited during the bio-decontamination phase), fans, an airtight bio-decontamination chamber, a heating element, a control unit for controlling all the decontamination cycle.

Inside the area 18, it is possible to place several series of products or objects to be decontaminated, for example on decks. It is, of course, possible to increase the number of loading and decontamination areas/lock devices, for example by superposition. It is thus possible to assign each of the loading areas with different bio-decontamination treatment durations. It is also possible to provide a removable separation between the two areas to then form an area of larger dimensions and/or of greater volume.

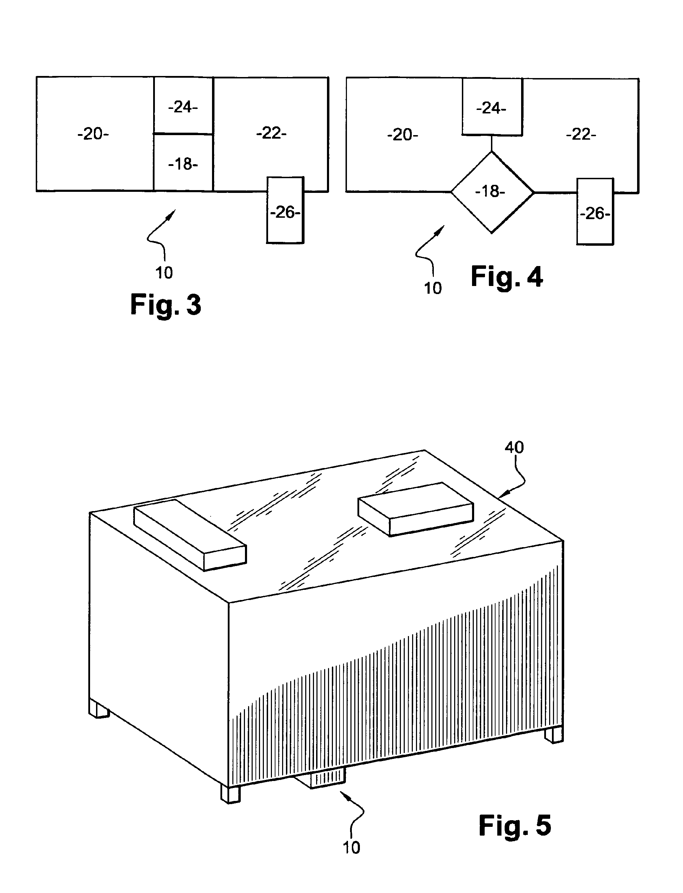

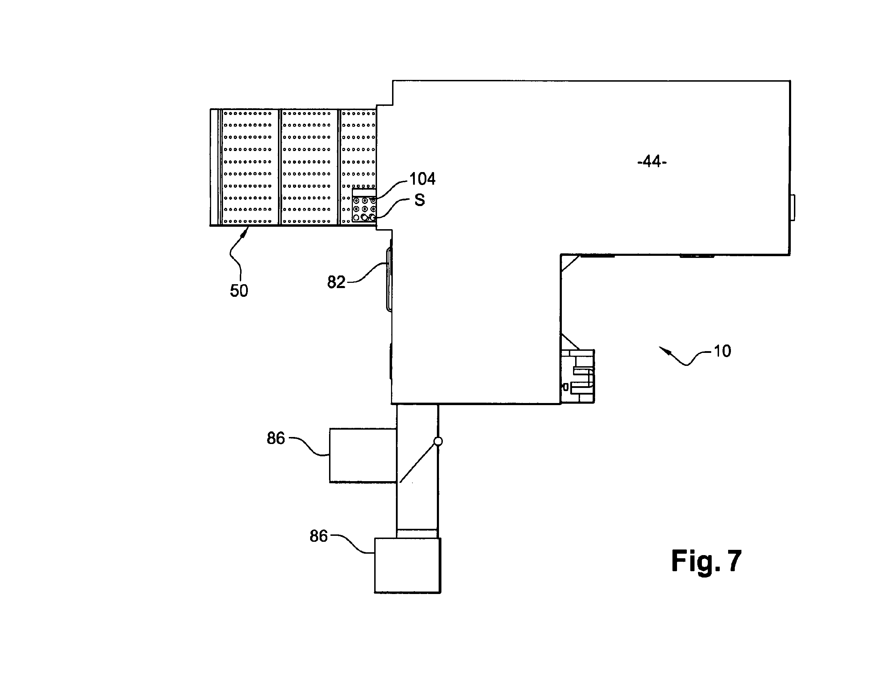

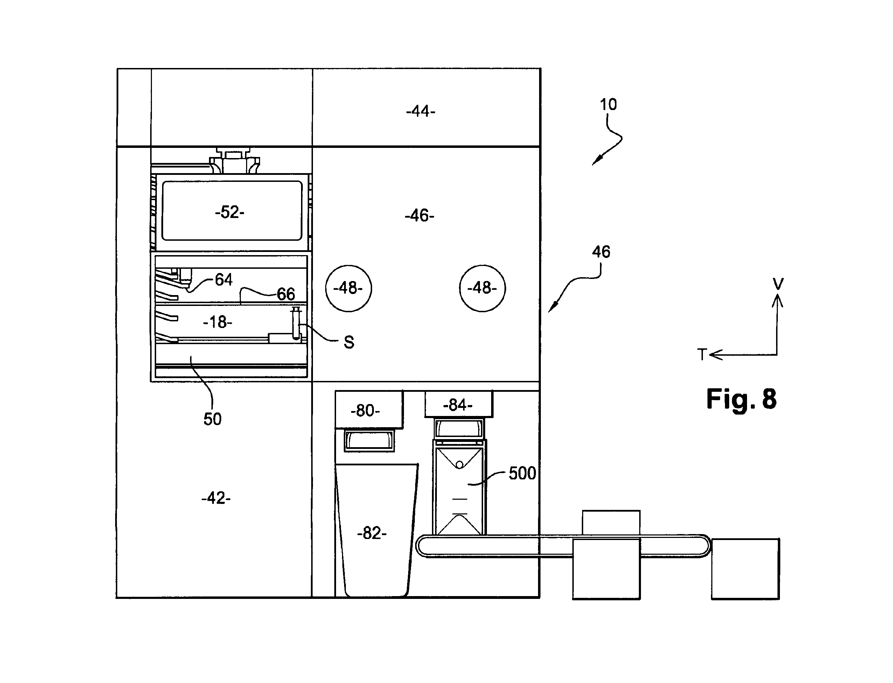

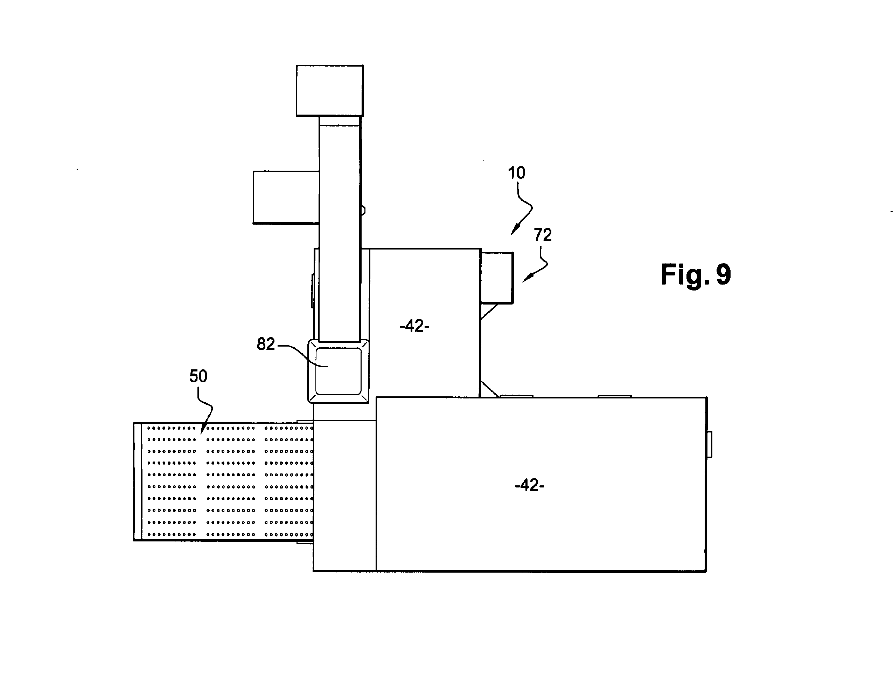

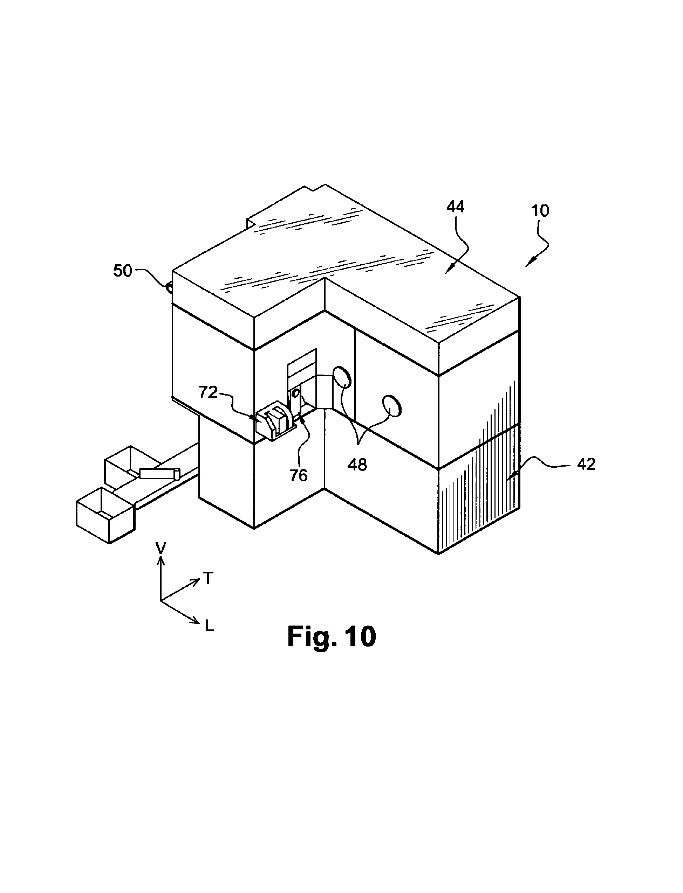

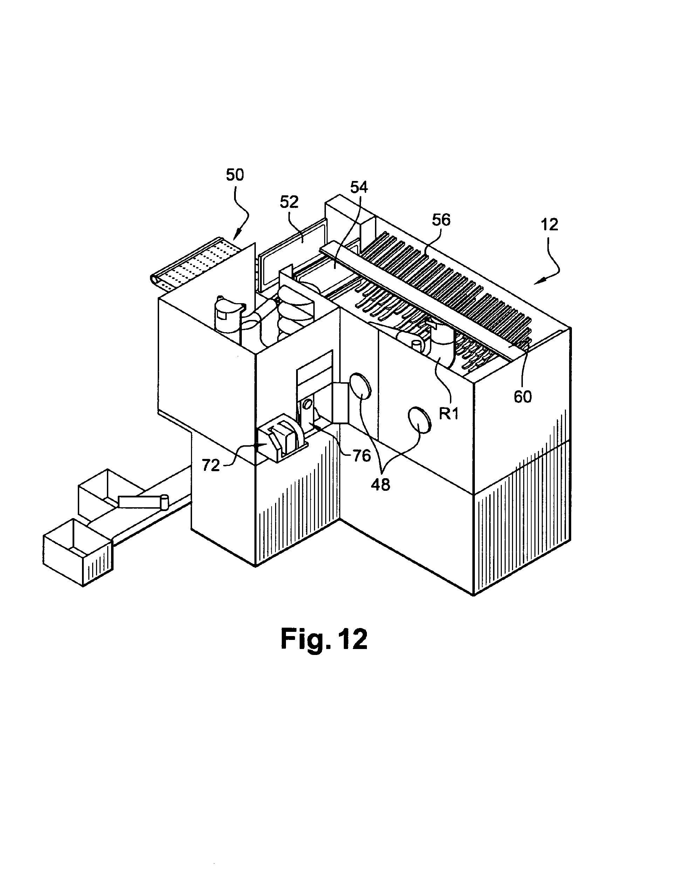

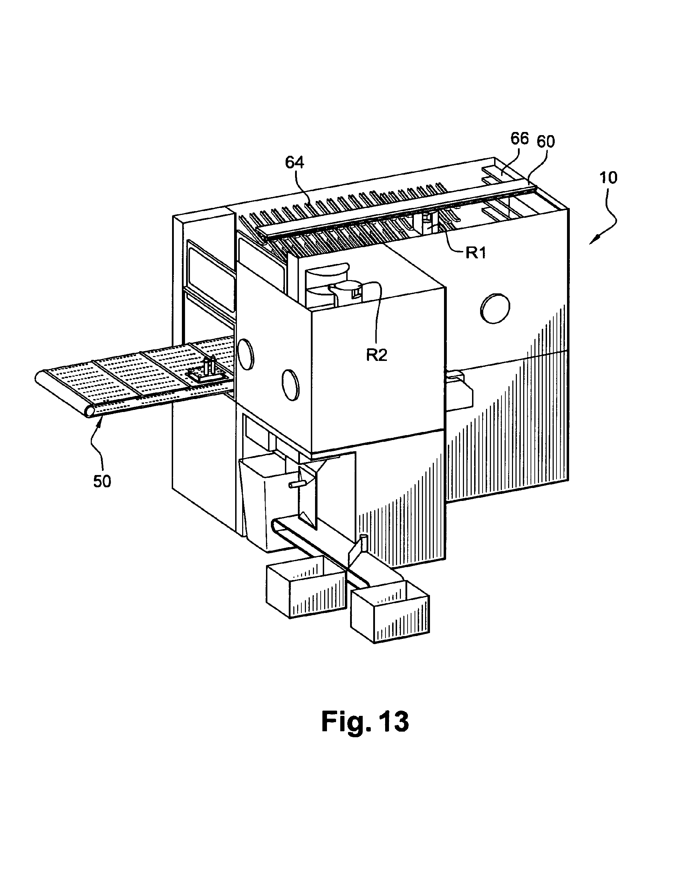

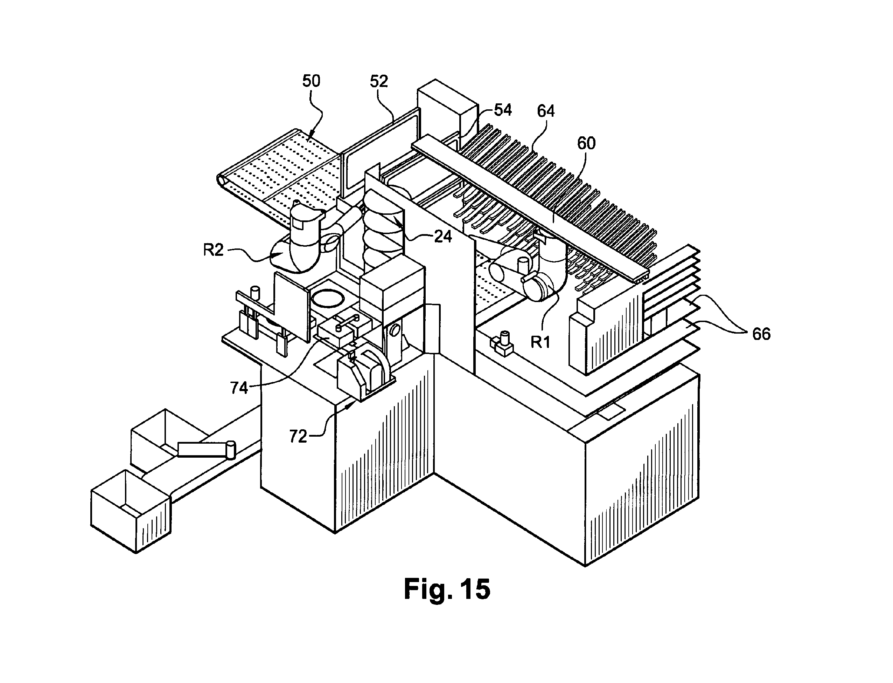

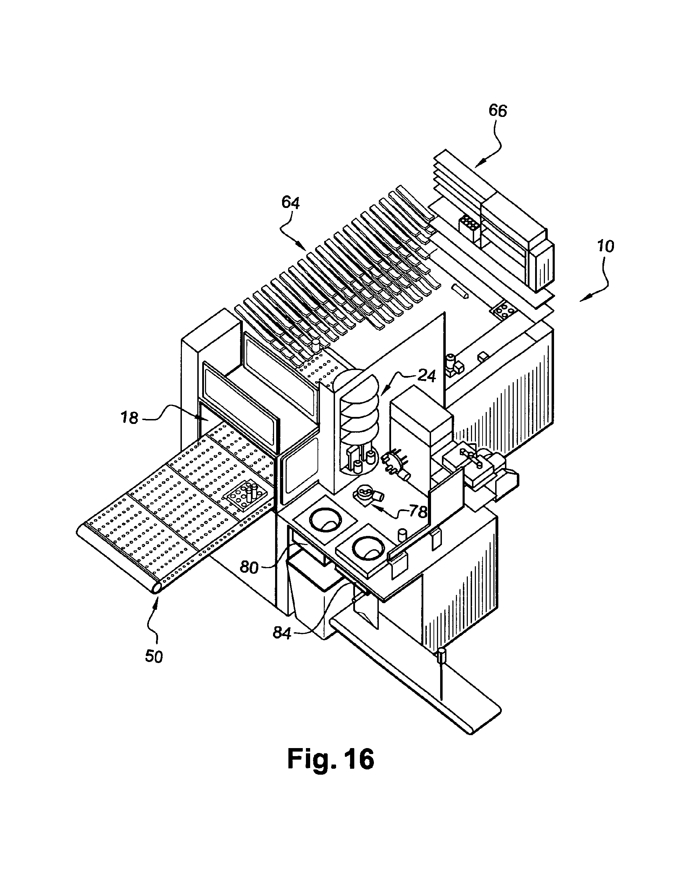

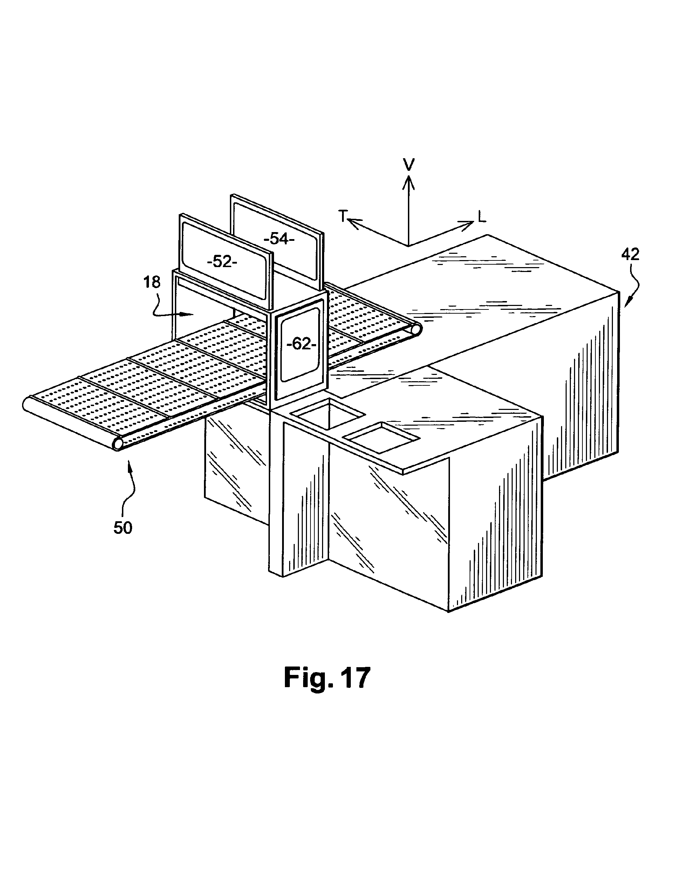

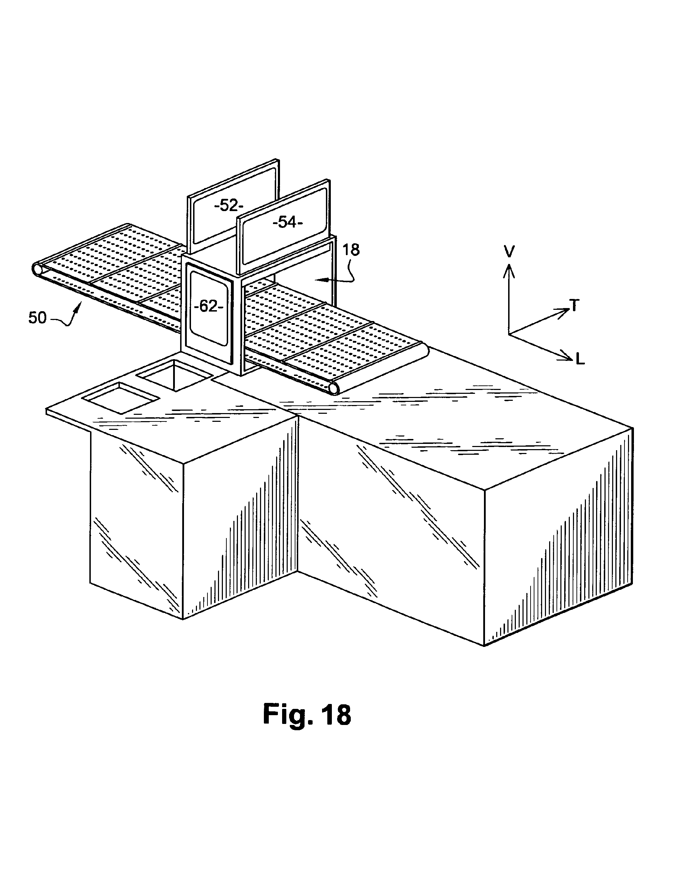

There now follows an exemplary and non-limiting description of an embodiment of an automated workstation according to the teachings of the present invention, more particularly concerning its general architecture, with reference to Figures 5 to 18.

In these figures, the terms Vertical, Transversal, Longitudinal, horizontal, etc., are used with reference to the L, V, T axis systems indicated in these figures.

The flexible tent 40 represented in Figure 5 is of generally rectangular parallelepipedic form, open in its bottom part and with certain vertical flexible lateral walls possibly being open.

Such a protective tent, with its own air filtration and circulation means, makes it possible to arrange the workstation 10 in a non-isolated room so as to satisfy the ISO class 5 to 7 isolation standards. The products are thus protected during loading and when they leave. Particles counters can be provided in the loading area under the tent.

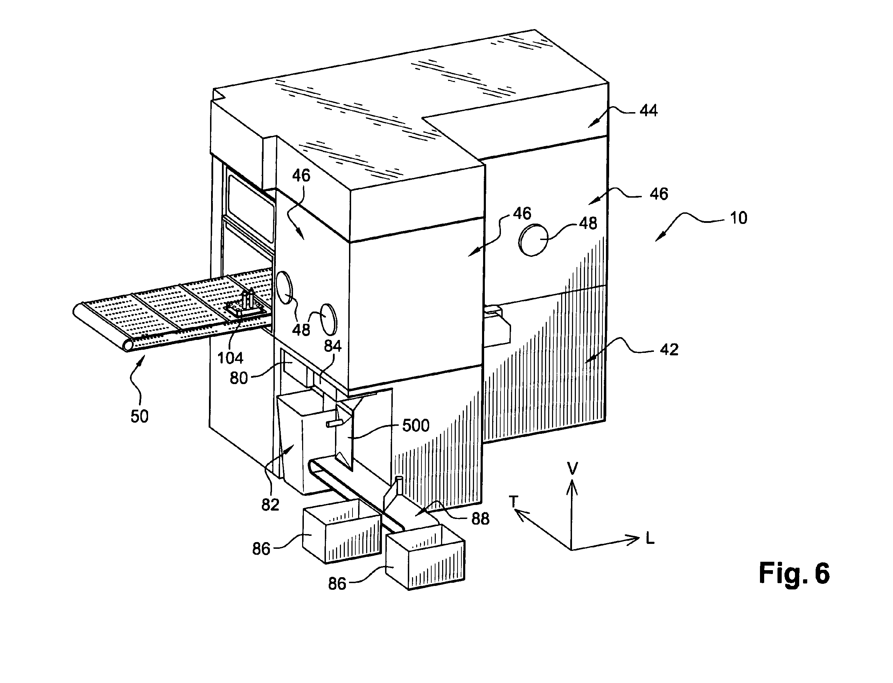

As it can be seen in Figures 6 to 10, the workstation 10 comprises a bottom part or base 42, in several assembled parts, a top roof element 44, and a central part, vertically intermediate between the base 42 and the roof 44, which is delimited laterally by vertical lateral walls 46, to form with the base 42 and the roof 44 an airtight chamber.

Some lateral wall elements 46 comprise, in a known manner, orifices 48 with integrated sealtight gloves. These gloves can be permanent or of DPTE - removable - type: the orifices are sealed by lids and the gloves are fitted only during non-ordinary handling operations (maintenance, cleaning, replacement of consumables and pipework).

These overall figures also show a horizontal loading deck 50, in this case in the form of a rolling mat, a front left portion of which projects longitudinally outside the sealed chamber of the station 10 and the other, rear part of which extends horizontally inside the chamber 16 to enable different elements and components to be introduced therein during loading phases, and this within the loading area 18.

Of course, the loading mat or mats of each lock device, or arranged otherwise in the station, are not necessarily a single piece but can comprise several parallel belts or equivalent, possibly controlled independently, for loading components and elements placed on these mats or belts at different loading speeds.

The loading area 18 is in this case a loading lock device with two moving vertical doors 52 and 54, controlled separately, the loading area 18 also being sealed transversally by other complementary vertical wall elements.

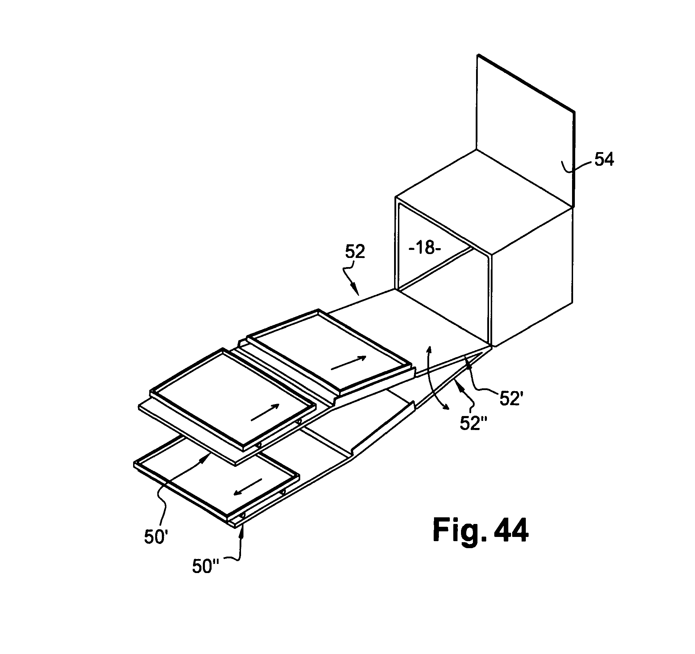

Figure 44 shows a variant embodiment of the loading lock device 18 which comprises a rear door 54 which moves vertically, but the front door of which, illustrated in two positions 25' and 52", is mounted to pivot alongside a transversal horizontal axis in the vicinity of the bottom of the loading lock device 18.

Facing the lock device 18, there are provided a top deck 50' and a bottom deck 50" in front of each of which the pivoting front door can occupy one or other of the two high 52' and low 52" positions, the door 52 of course also possibly being closed vertically to block the lock device 18.

The two decks 50' and 50" make it possible for one 50' to load into the lock device 18 when the door is in the position 52', and for the other 50" to recover empty decks and/or prepare other loadings.

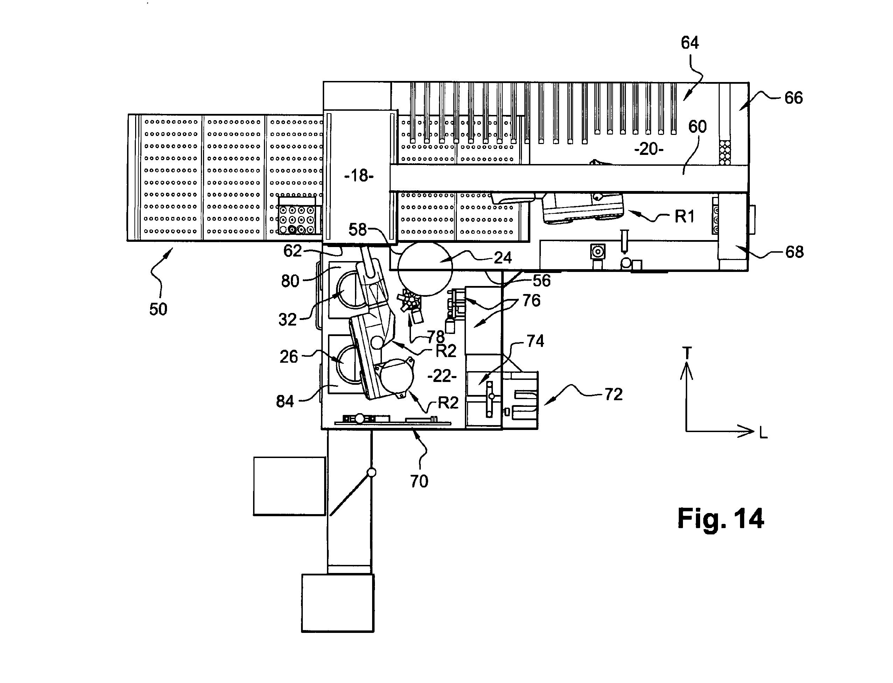

The plan view of Figure 14 shows the loading 18, storage 20 and preparation 22 areas, as well as the transfer area 24.

In this embodiment, the storage area 20 is separated from the preparation area 22 in particular by a longitudinal vertical partition 56 in which is provided a rotating vertical carrousel 58 with multiple shelving units making it possible to transfer different elements and components from the loading area 20 to the preparation area 22 and, where appropriate, vice versa.

A first articulated robot R1, in this case of the type with articulated arms with multiple axes, is arranged in the storage area 20 and it is mounted to slide longitudinally on a horizontal longitudinal top rail 60.

The area of activity of the robot R1 for handling different objects is thus the loading area 18, the storage area 20 and the transfer area 24 formed by the half of the carrousel 58 situated in this area through the wall 56.

A second robot R2, of the same type as R1, is arranged in a fixed manner in the preparation area 22 by being fixed to the roof element or to a top structural element.

The action area for the handling of different objects by this second robot R2 is the preparation area or volume 22 in which the robot R2 also has access to means 26 for removing and dispatching the final products, to the waste treatment part 32, and, possibly, to the loading area 18 if an opening, possibly controlled, is provided in the longitudinal vertical wall element 62 to enable communication between the loading area 18 and the preparation area 22.

The storage area 20 comprises in particular a multiplicity of shelving units and channels for the storage of vials, IV bags and syringes.

Inclined channels 64 are thus represented, for example for the storage of vials F, as are horizontal shelving units 66, some of which in this case belong to a refrigerated storage area 68.

The figures show, in the preparation area 22, a vertical support 70 serving as a support for the various preparation mechanisms and devices per se, with a view to producing mixtures from the contents of the vials for the preparation of final products which will be contained in syringes and/or intravenous flexible IV bags. There are also some shelving units in the preparation area for the containers of final products, for example before they are put together for a determined patient, as well as for temporary storage of vials and IV bags intended for use during preparation.

Also represented, arranged outside the station 10 but communicating in an airtight manner with the inside of the preparation area 22, is an analysis, inspection and validation unit 72, which is, for example, a Raman spectrometry unit. This unit makes it possible to check and verify, after its preparation and before its dispatch from the automated station 10, the composition of the final product.

Inside the preparation area 22, weighing means 74, liquid pumping means 76 and stirring and mixing means are provided.

In the preparation area 22, the bottom base 42 comprises two airtight disposal shafts one of which 80 opens out vertically towards the bottom outside the workstation so that the objects that are introduced vertically therein fall into a hermetic waste bin 82.

The other shaft 84 is for the removal of the containers, flexible IV bags or syringes, containing the prepared, packaged and labelled final products as will be explained herein below. The packages are then directed to different transportation and delivery boxes 86 by suitable conveying and/or pneumatic means 88 which are arranged vertically in line with the shaft 84 outside the workstation 10, under the corresponding part of the base.

The general method of preparing a final product can be summarized by the following phases: customer order → preparation of the necessary components and elements → sterilization/decontamination → identification/storage → reconstitution in a vial → labelling → preparation of the product → validation of the final product → packaging of the final product.

### Identification

Once the various components and elements have been loaded into the workstation 10 and once they have been decontaminated, they must be identified and verified before being stored on the shelving and similar units. Identification of the vials can be done by using a video recognition camera which will take and store an image of the label affixed to the vial.

The camera can also read any identifying barcode printed on the label. To read the label on the lateral face of the vial by means of the camera which is fixed in the workstation, the robot manipulates the vials to place them on a rotating table in front of the camera.

The IV bags are identified by using the same type of camera with the same capabilities. The robot places and holds the bag in front of the camera. The software of the computer system managing the installation as a whole and the workstation also makes it possible to read the inscriptions on the label and thus to interpret the appropriate nature of the medicinal substance and its concentration that must be used to prepare the prescribed dose prescribed through the pharmacy's computer management system.

After identification, and by means of the robot, the elements and components are stored in the different areas and storage means that are defined.

The types of movement and the sequence of movements of the robot to this end depend, of course, on the general architecture and the layout of the workstation, and in particular of the decontamination, storage and preparation areas. The storage area can be separated or not from the preparation area by different separation and/or communication means.

The classification and the storage of the products can be systematized in the form of decks placed on the shelving units, whereas the different components and elements can be handled individually by the robot and placed on flat or inclined shelving units.

Syringes: The station uses a variety of syringe sizes and colours. Three main sizes are for example used: 3 ml, 10 ml and 50 ml, but the station can use other types and sizes of syringes that are commercially available. The syringes are used to transfer the fluids from the IV bags to the vials, and also as containers of prepared final products.

The syringes are in particular fitted with a "Luer Lock" type end piece, suitable for secure connection with a swabbable valve. To add a diluent, it may be simpler to have standardized syringe tips or IV bags.

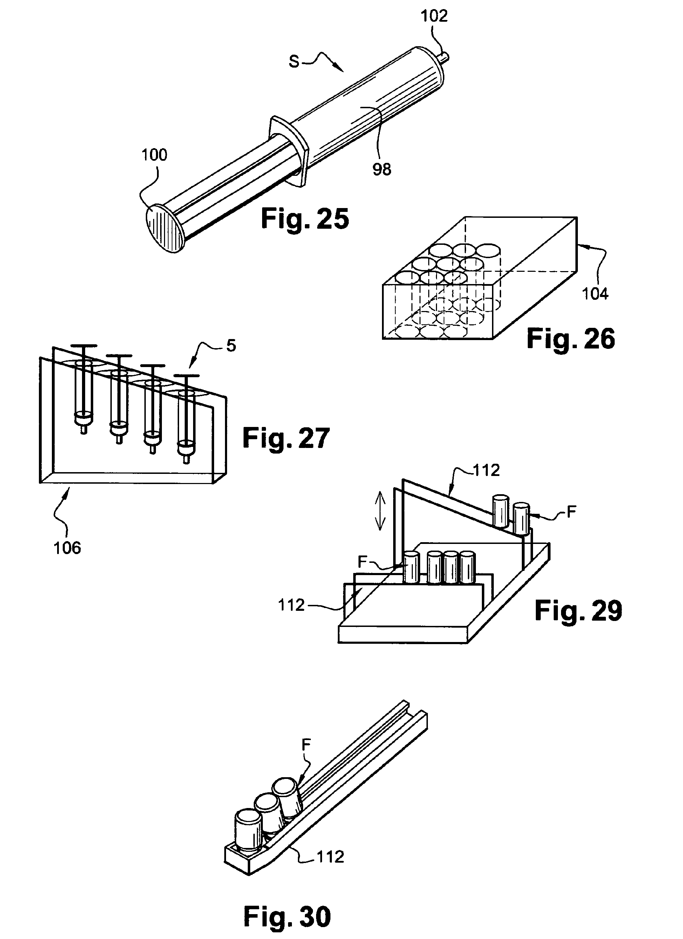

Figure 25 diagrammatically represents a standard syringe S comprising a body 98 and a piston 100 with a standardized tip 102.

### Loading of the syringes

Concerning the loading of the syringes or vials, there are various ways of proceeding in groups, for example by means of decks.

There are thus various possible ways of loading the syringes; either in a matrix deck, or by means of an inclined rail, or by using a special deck. As a variant, the possibilities can be combined to obtain another way of loading the syringes.

It is preferable for the syringes to be entirely packaged in an airtight manner during the bio-decontamination gassing operation. Thus, the matrix cell or deck will be closed in an airtight manner on the side and the lateral faces, for example with a thin plastic film. If the plastic film is removed in the loading area, ventilation must be provided above this area.

When the deck comprises a compartment for each syringe and the whole is sealed, this prevents all the syringes from being contaminated in case where one of the syringes leak. Consequently, each compartment can be opened to remove a single syringe, the others remaining protected. The opening of a compartment by cutting the plastic film in the top part can be performed by any means, such as a cutting laser for example.

When the syringes S are stored in a matrix deck, each syringe remains in the matrix until it is needed. The matrix is moved by the robot and it is attached to the wall or placed on a shelving unit.

When they are loaded on rails, the syringes remain on the rail and are taken by the robot and placed on the appropriate shelving unit(s).

Figure 26 diagrammatically illustrates a matrix deck 104 for loading the syringes.

Figure 27 diagrammatically illustrates an inclined rail 106 for loading the syringes S.

Vials for cytotoxic drugs: Cytotoxic drugs are mainly presented in two different forms, liquids and solids in powder form. They are fed into the station 10 in vials F of different sizes, generally made of glass; light or dark depending on the cytotoxic substance, and in particular its sensitivity to light.

The neck of all the vials and the top ring 94 in this case always have the same size, a diameter of 20 mm. The vial F forming an initial vessel may or may not be packaged in a hot-welded sheet. The vials are normally sealed by a capsule and packaged individually. The operator must remove the vial from its package before loading. The feature that is common to all the vials is in this case the shape and the size of the neck and of the top ring 94.

### Loading of the vials

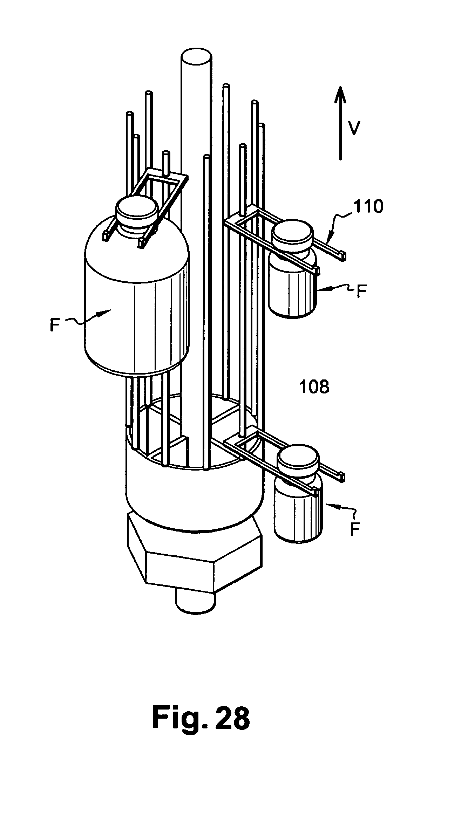

Figure 28 shows a loading basket 108 which can comprise a series of forks 110 grasping each vial F by its neck.

The solution illustrated in Figure 29 is a rail that is initially horizontal 112 in which the vials F are placed head down, the rail then being raised once placed in the workstation to enable the first vial to arrive in place at the end of the rail, automatically by gravity.

It is also possible to use a special inclined rail comprising a vialholding channel as illustrated in Figure 30.

Use of a filtered needle-less vial adapter and a closed needle-less device

It has been observed that risks of contamination occur because of the leaks and splashes that occur when a needle is removed from a syringe or a vial, and/or during degassing when a diluent is injected into a vial and the resulting pressure inside the sealed vial is released when a needle that is open to free air is inserted into the vial.

There are various closed needle-less transfer devices and systems, each of which mechanically prevents the transfer of environmental pollutants into the system and the escape of hazardous substances or vapour concentrations towards the outside of the system. They are used as secure devices for the preparation of antineoplastics by ensuring the sterility and non-dispersion of aerosols of antineoplastics.

The use of such devices and systems such as the "Tevadaptor" or "PhaSeal" (registered trade marks) is proposed in this case for the first time for the implementation of the automated station according to the invention for the preparation of hazardous products.

A filtered needle-less vial adapter B is fitted on each vial and remains fitted throughout the application of the preparation method.

The adapter, or pin B, is fitted on the neck of a vial to enable the content to be transferred to a sealed system, to withdraw a fluid/liquid substance for medical use contained in a vial.

The adapter, or adaptation pin, is able to be fitted to all vials with a 20 mm diameter neck. The adapter consists at one end of a small pin, and at the other end of a swabbable valve with Luer Lock tip, a locking mechanism suitable for a 20 mm diameter neck, and a 0.2 µm hydrophobic filter.

In the context of the method, the filter makes it possible, on the one hand, to equalize the pressures inside the vial to enable liquid to be added or removed more easily, and, on the other hand, to avoid the release of toxic particles into the atmosphere.

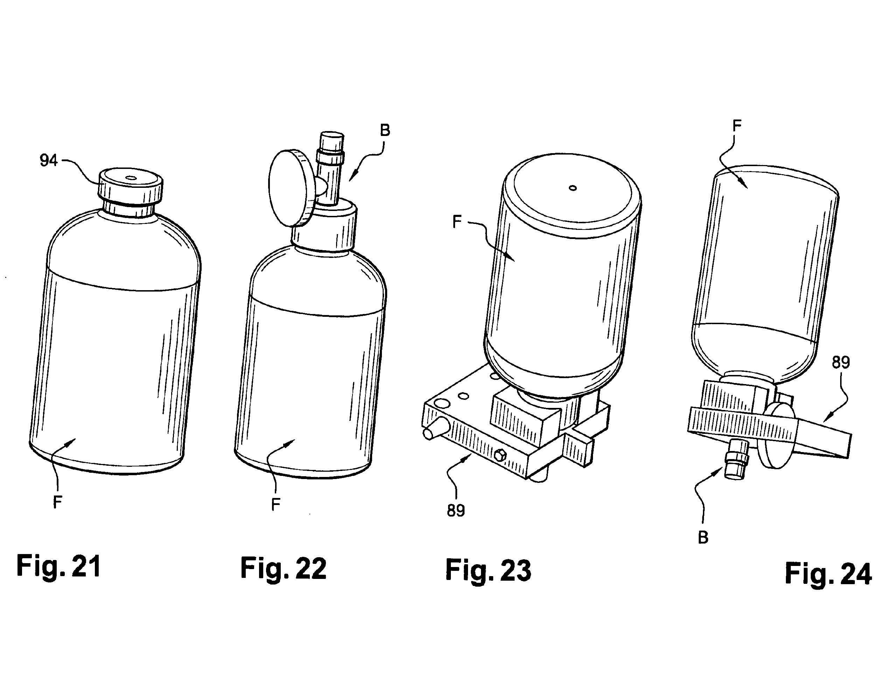

Figure 21 shows a vial F with its standard neck 94.

In Figure 22, the vial F is fitted with its pin B enabling use in a needle-less closed system, whereas Figures 23 and 24 represent two views illustrating the vial of Figure 22 in a position mounted on the universal adapter 89.

As it can be seen in Figure 24, the free end section of the pin B projects through the vertical through-hole 93 in the base 89.

Concerning the fitting of the pins B to the vials F, various possibilities are envisaged. The assembly can be carried out before the vials are loaded in the station, or even later, in a robotized manner inside the station.

For the loading and storage of the pins B, of commercially available models, the following elements can for example be used.

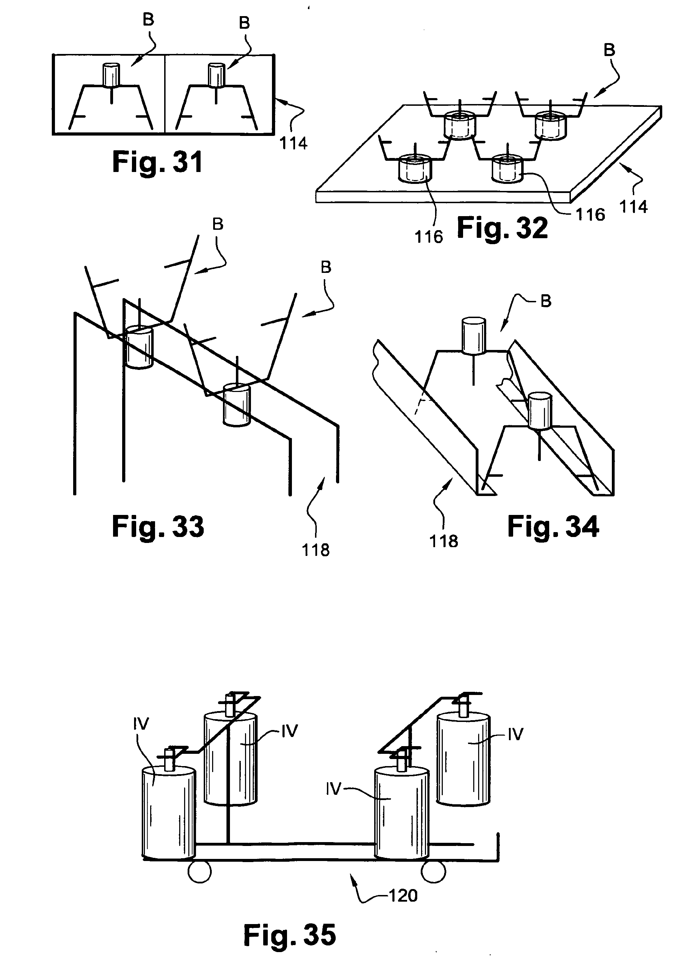

In Figure 31, as for the syringes, the pins B are housed in a matrix deck 114.

In Figure 32, the matrix deck 114 is a special deck with cavities 116, each of which receives, head down, a pin B.

Rails 118 such as those illustrated in Figures 33 and 34 can also be used.

### Closed needle-less transfer device:

A device for transferring substances for medical use which mechanically prevents the transfer of environmental pollutants into the system and hazardous substances or vapour concentrations from escaping outside of the system. It is, for example, a secure device for the preparation of antineoplastics ensuring the sterility and non-dispersion of antineoplastic aerosols.

Needle-less syringe adapter: This is an adapter which is adapted to a syringe with standard Luer Lock tip.

Luer Lock adapter: This is a component which makes it possible to convert a standard female Luer Lock connector (defined in the ISO 594 standard) into a sealed connector. It may also be used on an intravenous bag or IV bag Luer Lock. It prevents minor leaks and splashes.

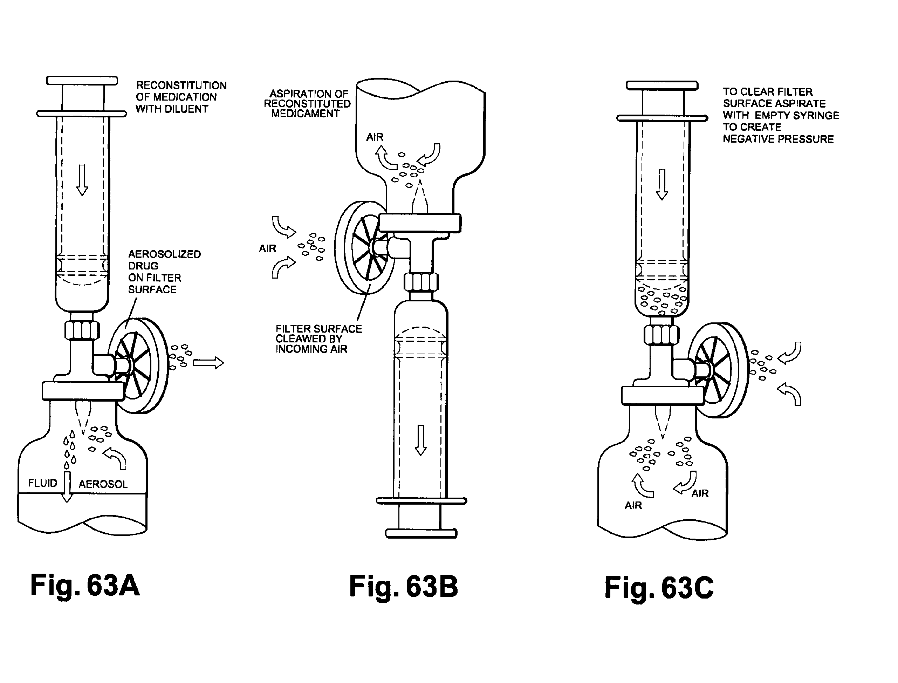

Figures 63A to 63B are diagrams illustrating an example of known means that are available in the state of the art to form closed needle-less systems for connection between a vial and a syringe, by means of a pin with filtration means.

### IntraVenous IV bags

A wide variety of IV bags can be used in the workstation. The sizes of IV bags vary from 100 ml to 1000 ml. The contents of the IV bags vary between, for example, sterilized water for injection, sodium chloride or dextrose solutions.

The workstation preferably uses "MacoPharma IV" bags which are fitted with a swabbable valve system or any other commercially available closed needle-less system. The IV bags are used for the dilution, the recovery of liquid waste, or as containers of prepared final products.

The workstation is designed to use all types of IV bags regardless of their sizes, shapes or manufacturers.

### Loading the IV bags

Because of the size and the flexible nature of the bags, it may be necessary to use different kinds of "constraining" structures so as to be able to move the bags during the various steps of the method. These structures can cover various forms and be of various designs.

Such a support structure can be mounted on the associated bag outside the workstation 10 by an operator, or inside the storage area by the robot.

Figure 35 is a diagram which illustrates a simple support structure 120 bearing IV bags without requiring any adaptation means to be fitted, the IV bags being simply suspended.

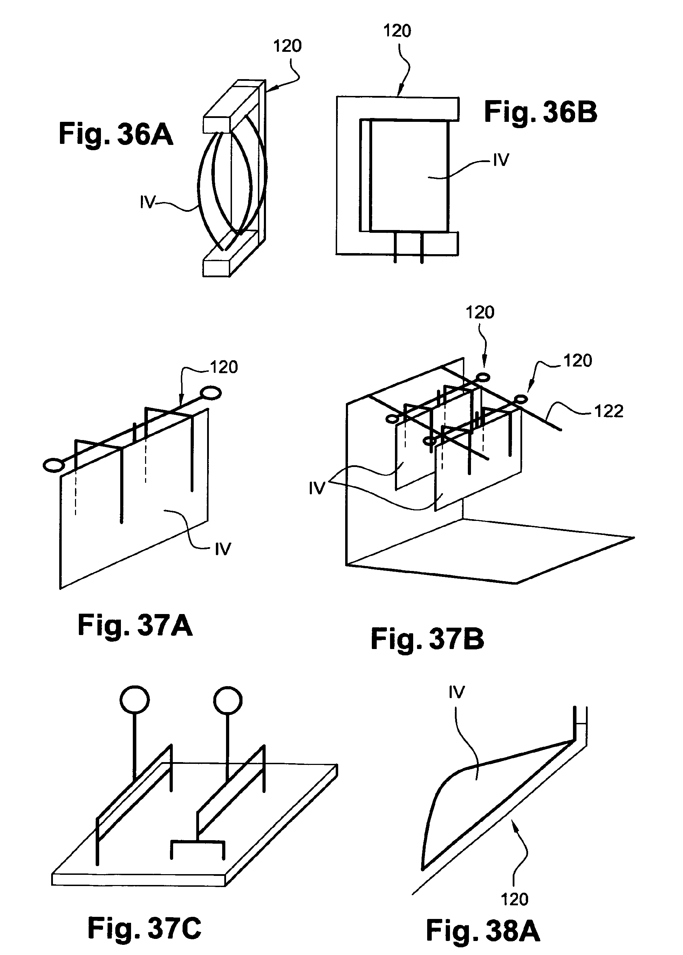

In Figures 36A and 36B, the structure 120 is a C-shaped frame or clamp in which the top edge of the bag and its tips are clamped.

Figure 37A shows a support structure 120 with inverted U-shaped double adapter and Figure 38A shows an inclined structure 120 on which the IV bag rests by gravity.

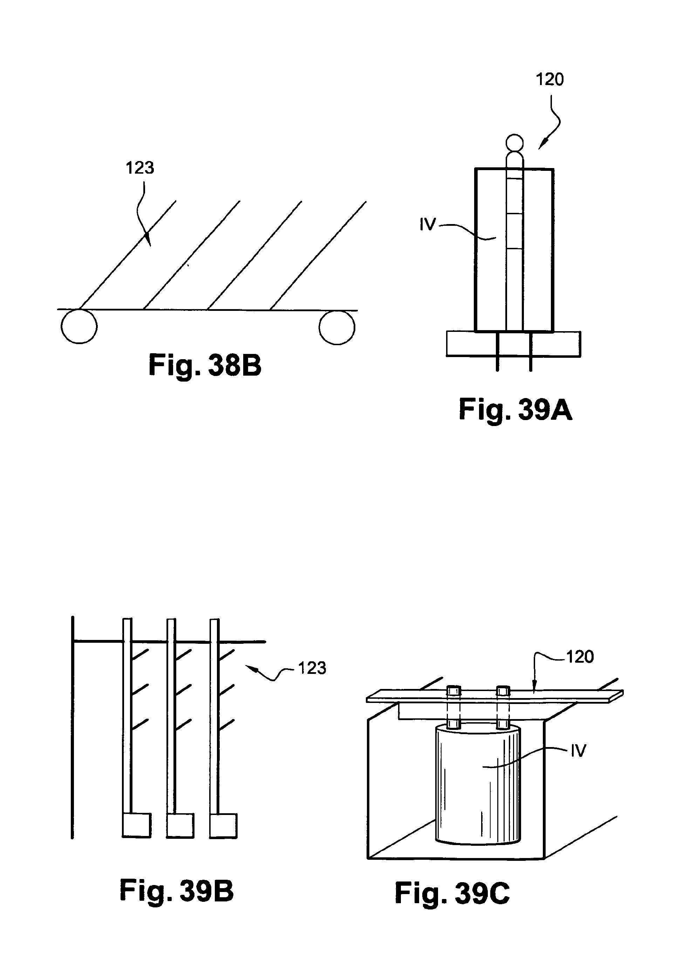

Figure 39A shows a T-shaped support or structure 120 and a variant of the latter is shown in Figure 39C.

In all cases, the robot has the capability to detach the bag from its structure 120 or to perform the reverse operation.

The structure 120 for adaptation to an IV bag is designed to enable the bag to be detached by the robot, a bag to be transported vertically, or head down, and several bags at a time to be transported and positioned on a shelving unit.

For loading, the solution illustrated in Figures 36A and 36B requires nothing more than the simple placement on a deck. The solution 37A requires the use of suspended rails diagrammatically represented in Figure 37B and designated 122.

Inclined structures 120 of Figure 38A are stored on a special deck illustrated in Figure 38B with inclined support plates.

The solution illustrated in Figure 39A uses special decks 124 with positioning holes illustrated in Figures 39B and 39C.

Regarding the IV bags, there are four dedicated rail systems for storing them.

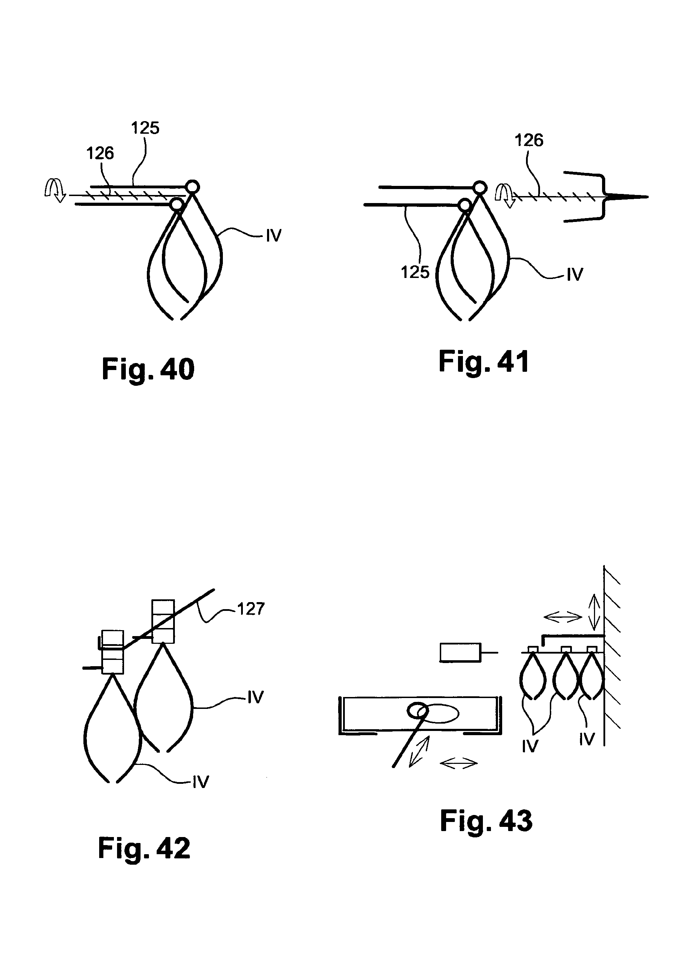

Figure 40 shows a channel 125 with a drive screw 126 for loading or removing the IV bags.

In Figure 41, the screw 126 is installed on the head of the robot.

Figure 42 shows an inclined rail 127 and Figure 43 shows a smooth channel system with a moving rod which can be installed on the wall or on the robot.

When an IV bag is fitted with a tube or a set of tubes, called neutral line, the (needle-less) filling port or tip is isolated and maintained inside the same clamp or support structure of the bag, thus enabling a syringe to be filled with the diluent and this diluent to be injected into the neutral line tip so that the neutral line is filled with a fluid containing no drug or medicine. This enables the nurse to connect the neutral line to the catheter of a patient without being exposed to droplets or splashes of drug or toxic product which can be produced when the intravenous neutral line is primed.

The components and elements such as the vials, the pins, the syringes, etc., can also be loaded by simply positioning them on a deck or a mat and they are then identified and recognized inside the installation by image processing means, each component or element then being taken by the robot and placed on its corresponding shelving units or inclined rails.

Once the various filled components have been identified, they must be placed in the classification and shelving unit systems provided for this purpose in the storage area, and the position of which depends directly on the manner according to which the components and elements have been filled inside the station.

For the vials and the pins, similarly, if they are arranged in a matrix, the matrix arrangement is retained, as for the positioning with rails.

### Universal handling support

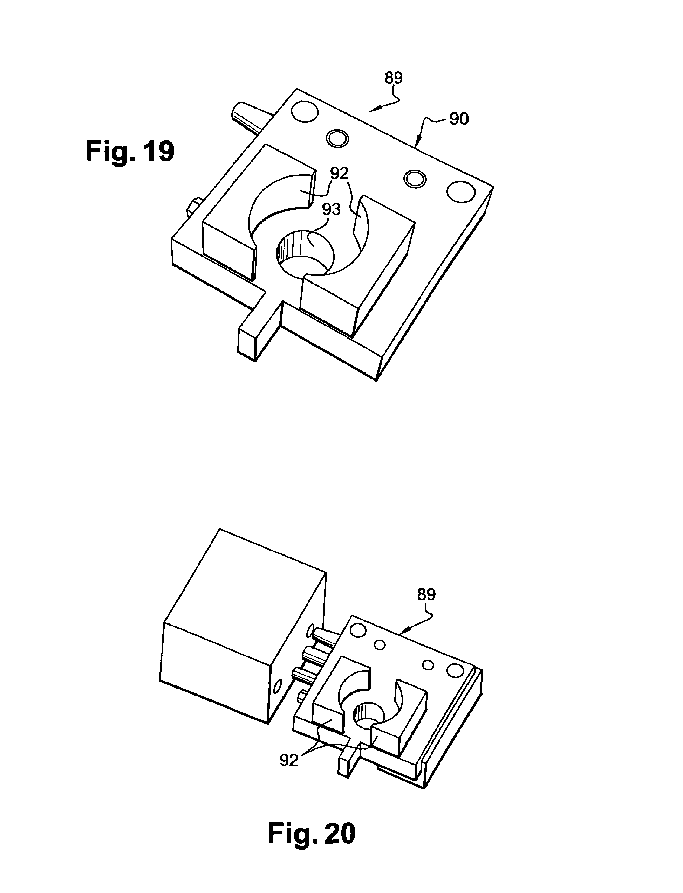

Figures 19 and 20 show a universal handling support particularly for the grasping and handling of vials, regardless of the vial dimensions, and for the handling of IV bags.

The support 89 in this case mainly comprises a base 90 on which may be mounted adjustable jaws 92, controlled to be driven to clamp and unclamp around the top part fitted with an adapter or pin B. The base 90 common to all the applications comprises, laterally, means adapted to the gripping head of the robot or of another gripping and handling device or mechanism. A small vertical lateral face of the base 90 can include a label identifying each determined universal support, for example of barcode type, in order to enable the computerized processing and certain identification of what is supported by the support.

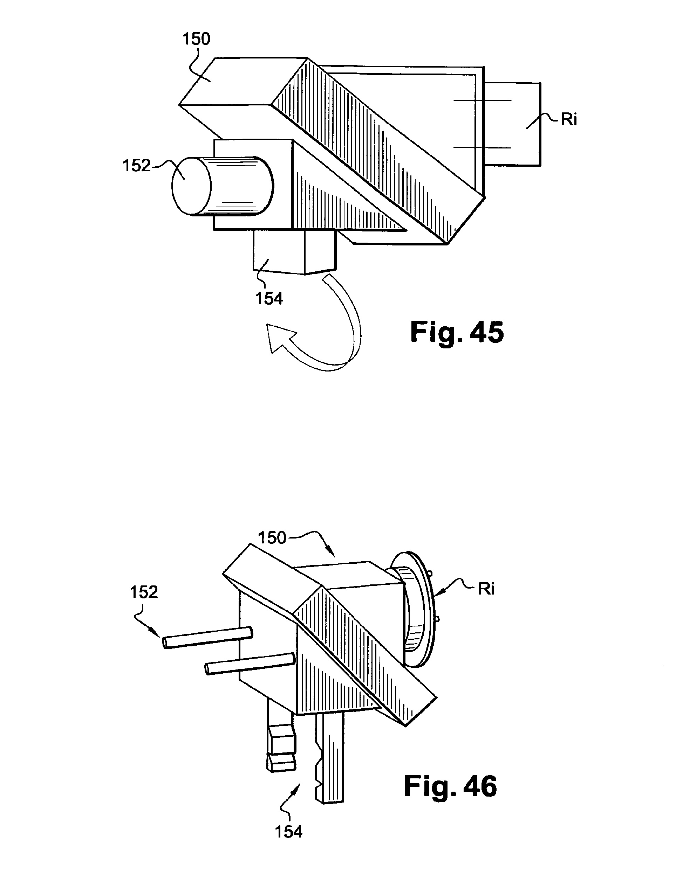

The free end or grasping head of the handling robot can include means suitable for simultaneously gripping several universal supports, whether they bear vials and/or bags, in order to move them simultaneously. These means can, for example, be long rods which pass through horizontal through-holes provided in the bases (see Figure 46, reference 152).

Concerning the universal support 89 with jaws 92, the latter can be motorized to be moved in both directions (opening or closing), but they can also preferably be returned permanently elastically in the direction corresponding to their closure and clamping so that the robot, when it wants to remove the adapter 89, separates the two jaws from each other against elastic return means.

As can be seen in the diagram of Figure 45 and in the perspective representation of Figure 46, the free end of the arm of a robot R can be fitted with a pivoting head 100 so as to be able, for example, to occupy two functional positions of use by pivoting according to the arrow in Figure 45.

The head 150 is also fitted with two "tools", namely the gripper 152 with rods that can be introduced into corresponding holes of the universal supports 89 and, for example, a clamp 154 able to take other elements by direct clamping.

### Transfer from the storage area to the preparation area

When the machine prepares a determined dose for a patient, the necessary objects, products, components and elements are moved into the preparation area once the preliminary phases have all been completed.

The following are then likely to be transferred, for example: an IV bag with its adapter, a syringe with a closed needle-less syringe system, a vial with its adapter borne by a universal vial support.

When the machine is not in the process of preparing one or more doses for patients, the station can transfer other elements such as a pollutant leak and splash recovery mat, all appropriate cleaning products, or all necessary tool parts. All these elements need to be transferred from the storage area into the preparation area through transfer means which can be of the following type.

Mouse hole: this is a simple hole of small dimensions provided between the two areas; the mouse hole can be fitted with a belt conveyor and/or a hatch with a flap, the hatch comprising a sliding door which drops if noxious particles have been detected that correspond to high levels of contamination or spillage.

The transfer means can also consist of a transfer lock device with or without doors as described previously.

It is possible to use transfer means similar to the introduction lock device with gas-based rapid decontamination means, meaning that there is no direct connection between the storage area and the preparation area.

Multiple and distinct transfer means can be provided, each assigned to transfer a product or component type, for example, one passage for the vials, one passage for the syringes, and one passage for the IV bags.

It can even be a tunnel with a rail and carriage system for the IV bags and the vials.

In the exemplary embodiment described previously and represented in the figures, it is a carrousel 58 or rotating door with two opposing faces. The shelving units situated on each of the two sides can be filled so that the rotation of the carrousel provokes the mutual transfer from one side to the other of the corresponding elements borne by the shelving units.

Each of the opposite faces either side of the vertical plate of the carrousel forming a revolving door closing the rectangular opening provided in the partition, can be fitted, not with shelving units, but with a preparation wheel 300 of the type that will be described later.

As for the shelving units, while the robot inside the storage area fills the wheel inside the storage area, the mixing, dilution and preparation operations are performed by means of the other wheel arranged on the other side of the wall or revolving door, that is, on the side situated in the preparation area.

### Preparation

Generally, the preparation of a final product can be carried out in three ways.

Firstly, different units can be used to perform the dilution then the mixing and weighing, and finally the preparation proper in a preparation unit.

Secondly, by using a wheel system which will be described hereinbelow, the same wheel unit is used for the dilution, mixing and preparation proper.

Thirdly, it is possible to use a peristaltic pump for dilution, followed by mixing and weighing means 74, and finally a dedicated preparation unit.

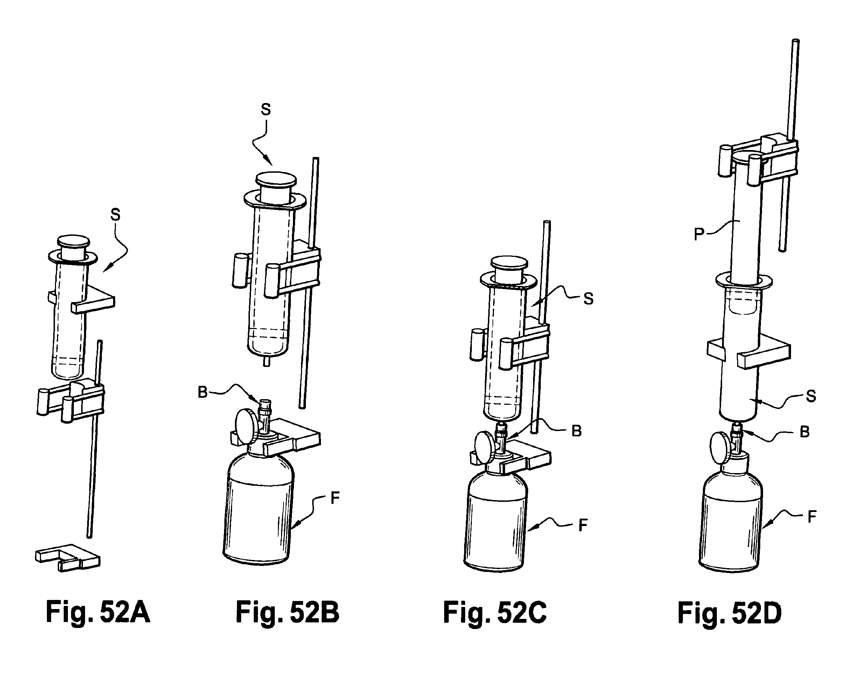

As diagrammatically illustrated in Figures 52A to 52D, and by means of a simple robot or programmable logic controller with three axes, it is possible to perform the four main operations consisting in adding diluents to the vial F, verifying the weight of the quantity of diluent added to the vial, mixing the powder and diluents, and transferring the liquid with the medicinal substances from the vial into the container of the final product such as the syringe S.

The first operation represented in Figure 52A is the grasping of a syringe S.

Figure 52B shows the syringe S aligned facing the pin B of a vial F.

In Figure 52C, the syringe S with its appropriate tip is "connected" to the pin B to form the closed system.

In Figure 52D, by acting on the piston P of the syringe S, upward or downward, liquid can be extracted from and/or introduced into the vial.

When the cytotoxic product is in liquid form, the method is reduced to a simple operation which is the transfer of the liquid from the vial into the final container.

Furthermore, diluents can be added before extracting the cytotoxic product in liquid form.

If the final container is an intravenous IV bag, it may be necessary to take some liquid out of the bag before adding the appropriate quantity of cytotoxic drug.

It is of course possible to turn over all the components in order for the vials to be in the high position, which provides a way of guaranteeing the extreme accuracy of the quantities of liquid extracted from the vial using the needle-less system.

Means similar to those diagrammatically represented in Figures 52A and 52D are incorporated in the preparation unit of the workstation described previously.

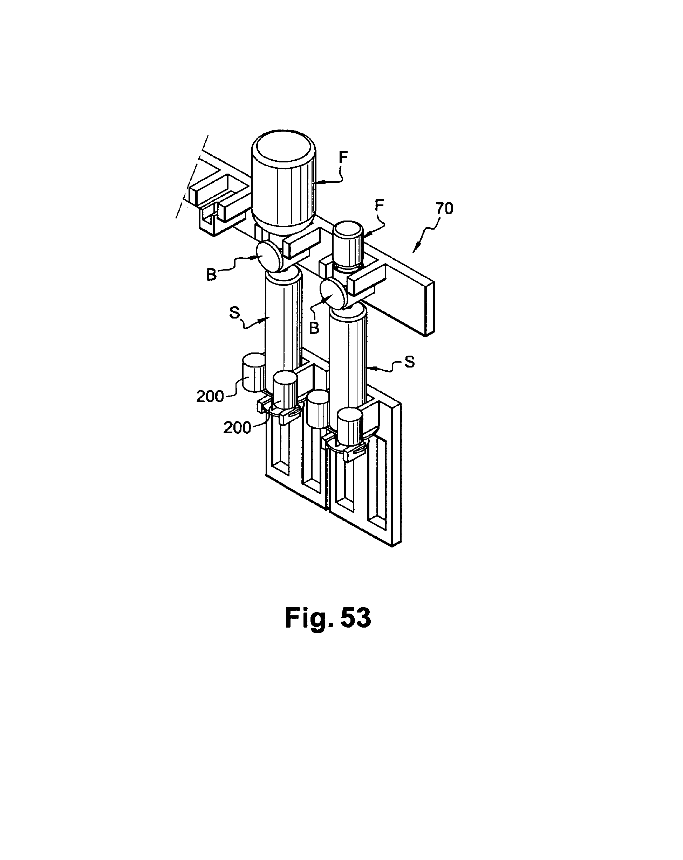

These means 70 are partly represented in Figure 53.

Also represented in this figure are motorized means 200 for driving the syringes rotation-wise, particularly to enable them to be connected by screwing their tip to a complementary system, in particular to a connection for an IV bag.

### Preparation by means of a preparation wheel

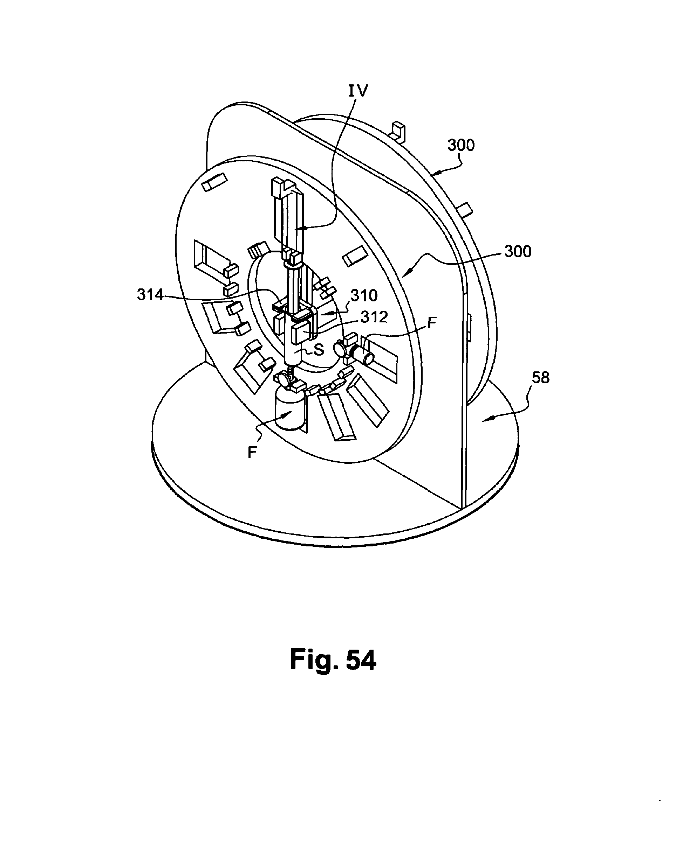

Figure 54 shows a carrousel with two wheels 300 of similar design.

The presence of two wheels 300 is not mandatory and the carrousel described previously can be retained for the transfer. It is possible to provide a single wheel 300 which will now be described in detail, used in particular as a preparation wheel.

The wheel comprises an internal device 310 with a clamp 312 which cooperates with the body of the syringe S, which holds the syringe and which can enable the syringe to be moved from top to bottom while the Luer Lock tip of the syringe is in communication with or connected to the valve of the IV bag or the pin B of a vial F.

A second clamp set 314 holds the syringe in place while the fluid(s) are being transferred. This second set is optional. The syringe needs to be able to be driven in a screwing movement to finish the connection and adaptation to a vial or an IV bag.

When the syringe is completely connected, the internal device holds the syringe S during the fluid transfer operation. The device also pulls on the piston of the syringe to extract diluents from the IV bag and pushes the piston so as to inject the diluents into the vial F, in this case in the bottom position. Of course, to switch from one to the other, the device 310 performs, taking Figure 54 as the model, a rotation of 180° about its horizontal axis.

As a variant, the external wheel revolves relative to the device 310. Here again, at the moment when the liquids are being transferred, the vial F can be in the high position relative to the syringe.

When the syringe is filled with the diluents, the internal device which holds the syringe revolves from top to bottom to inject the diluents into the vials after the syringe has been adapted and screwed to the pin B of the vial. The vials and bags are placed on the wheel by the robot. Each bag is held in position on the wheel by suitable clamps.

The annular periphery of the wheel can receive, in addition to the vials and/or IV bags, other syringes.

The outer ring of the wheel 300 can also be used as a mixer by driving it rotation-wise in both directions.

Thanks to the wheel, the fact that the bag, the vial and/or a syringe into which or from which the liquid is introduced or extracted can still be placed in the top position guarantees a very high accuracy of the volumes exchanged with the needle-less devices.

The mixing can be handled by the wheel, the stirring mixer

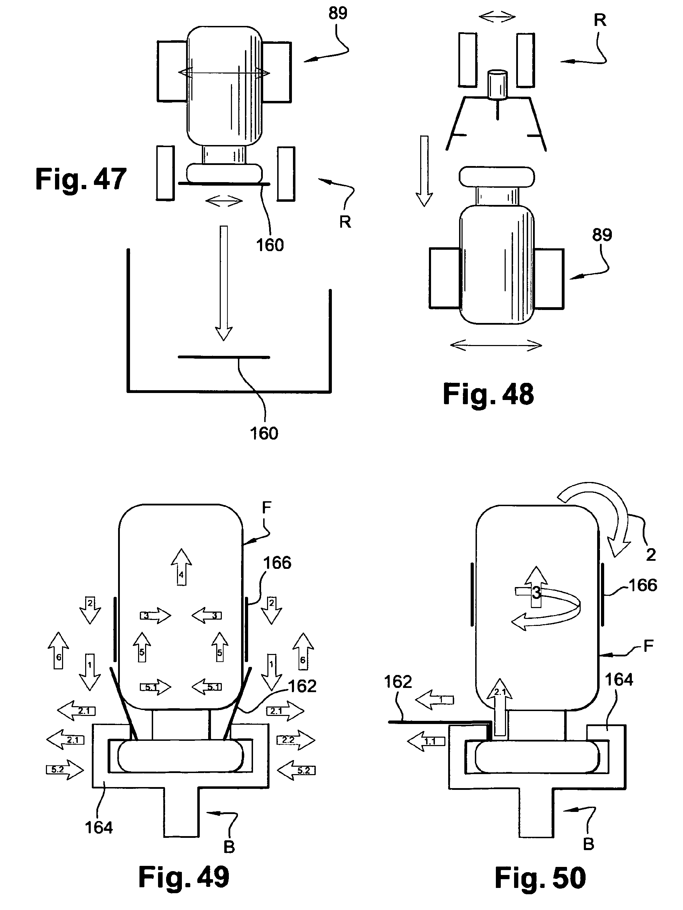

Figure 47 diagrammatically represents the procedure for removing the cover of a vial F. The vial F is fitted, for example, with a simple cover or cap made of plastic 160, using the same unit, that is the same technical means, used to mount a pin on the neck of the vial. It is also possible to use the robot and/or these means. The vial F is provided with its adapter or clamp 89 and the robot R is used to remove the cover 160.

Similarly, Figure 48 diagrammatically represents the mounting of a pin B using the same robot R on the neck of the vial F. It has a linear movement from bottom to top of the pin B relative to the vial F as indicated by the arrow. The vial F, to this end, is held by the gripping means 89. The result is thus the preparation of a "kit" or subassembly consisting of a vial F without its cover and fitted with a pin B.

When a vial F is empty, it may be desirable, for example with a view to the reuse and/or the reuse of the pin B, to detach the pin B from the vial. To remove the pin B from the vial F, specific means for this purpose must be provided. It is possible either to push on the clip attaching the pin to the vial and pull the vial upward, as diagrammatically represented in Figure 49, or tilt the vial to one side and "unscrew" the vial, as diagrammatically represented in Figure 50.

In Figure 49, a part 162 is introduced between the vial F and the clip 164 of the pin B, everything in a vertical position. A moving clamp 166 slides between the vial F and the part 162 by pushing it partly horizontally so as to separate the clip 164. The clamp 166 clamps the vial F sufficiently to hold it, but not sufficiently for the clip 164 to once again trap the vial. The clamp 166 holds the vial and pulls it upward. As the part 162 rises, there is less pressure to be applied and the clip is released. It is then possible to remove the part 162 so as to release the clip 164 for another use.

In Figure 50, the part 162 slides between the vial and the clip 164. The part 162 is moved from one side only, which opens the clip 164 on one side only. The clamp or jaw 166 holds and tilts the vial on its side which is still held in the clip opposite to the part 162. The jaw 166 then rotates the vial to extract it from the clip 164.

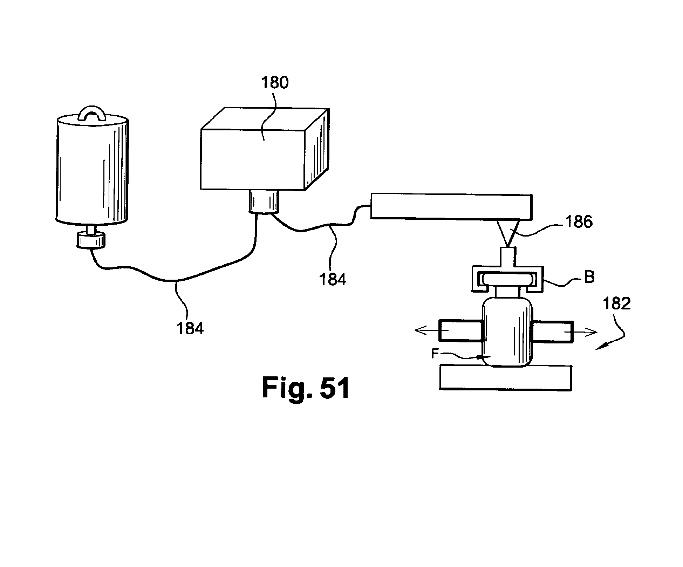

As can be seen in Figure 51, the pumping unit consists of three sections, a pump 180 per se, the means 182 for supporting and holding the vial F and tubes 184 linking the pump to a bag and to a moving needle 186 for filling the vial. The pump can be held outside the preparation area in a portion of the ISO class 5 standard installation that will be accessible to a technician to change the filling bags. It could also be positioned inside the preparation area. In this case, the pump can be reconditioned and the pipes and bags changed each morning.

The filling function 182, 186 of the vial F is in the preparation area and means for placing and feeding the vial are provided for its positioning with its pin B relative to the needle 186. The means for aligning the vial F can also then be used to shake it for mixing purposes.

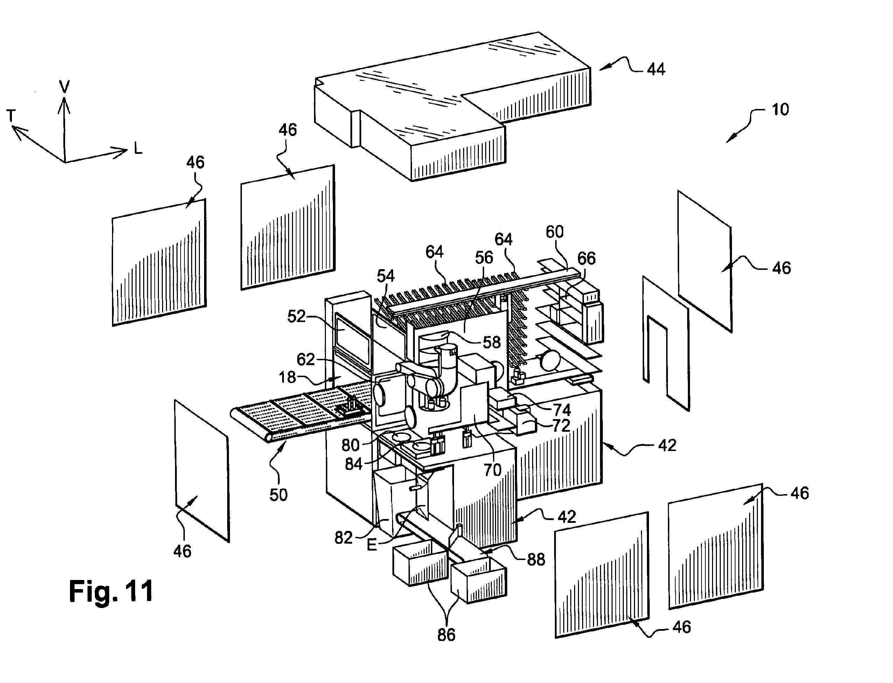

To verify the weight in particular of the vials, weighing means such as a balance 190 are provided in the preparation area (see Figure 11). The other stirring and mixing means 78 illustrated in the figures, in particular in Figure 14, there is a drum with vertical axis on which the vials and/or syringes are positioned radially with their axes horizontal. The vials or other items locked on the drum are driven in alternating rotation.

### Labelling the final product containers

The final product containers come in two forms, syringes or IV bags, these two forms being in ranges of different sizes.

The information printed on a label intended to be adapted to a syringe or a bag can be as follows: name of patient, preparation date, name of final product, dosage or concentration, and a barcode for automatic traceability.

The contents of the label are supplied by the computer processing system. It is also possible to add, to the label affixed to the bag or the syringe, other information, but these labels are generally small. The information may be storage instructions, expiration date, patient administration instructions, and the identification and geographic location of the final destination of the container, etc.

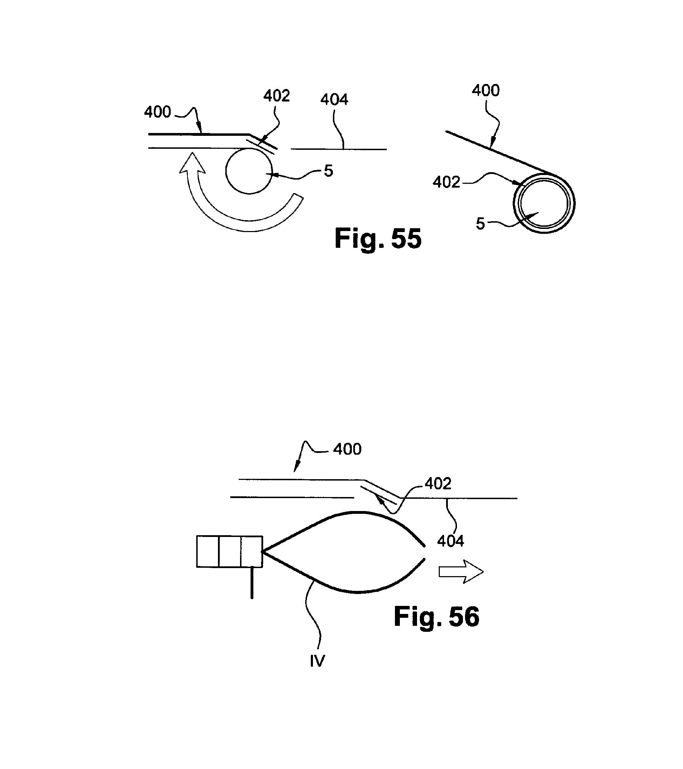

Two theoretical examples for affixing a label to a syringe and to a bag are illustrated in Figures 55 and 56.

In Figure 55, a label 400 is affixed to the external cylindrical surface of the body of the syringe S by winding it around the latter with the addition of an adhesive 402.

It is possible, for example, to rotate the body of the syringe relative to the label.

As can be seen in Figure 32, to glue the label 400, a relative horizontal displacement of the IV bag in relation to the label 400 and its adhesive 402 under a support 404 is applied, which makes it possible to compress the label onto the bag.

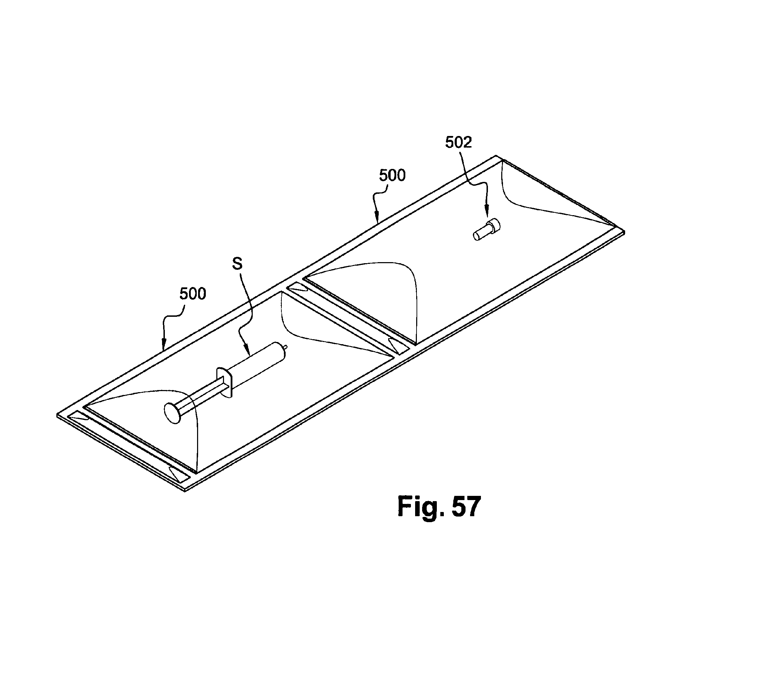

The final product containers are, before exiting the workstation, all packaged in envelopes, pouches or sachets 500 as illustrated in Figure 57. The sachets 500 are transparent so that their contents can be seen.

Figure 57 shows a double sachet 500, the main part of which, on the left, in this case contains a syringe S filled with the required quantity of final product, whereas the other part, on the right, contains a small vessel containing a sample of the final product contained in the syringe S. The sample 502 associated with the syringe S can then be used for verifications or complementary tests, in particular before the administration of the product, or be used for subsequent verifications after administration.

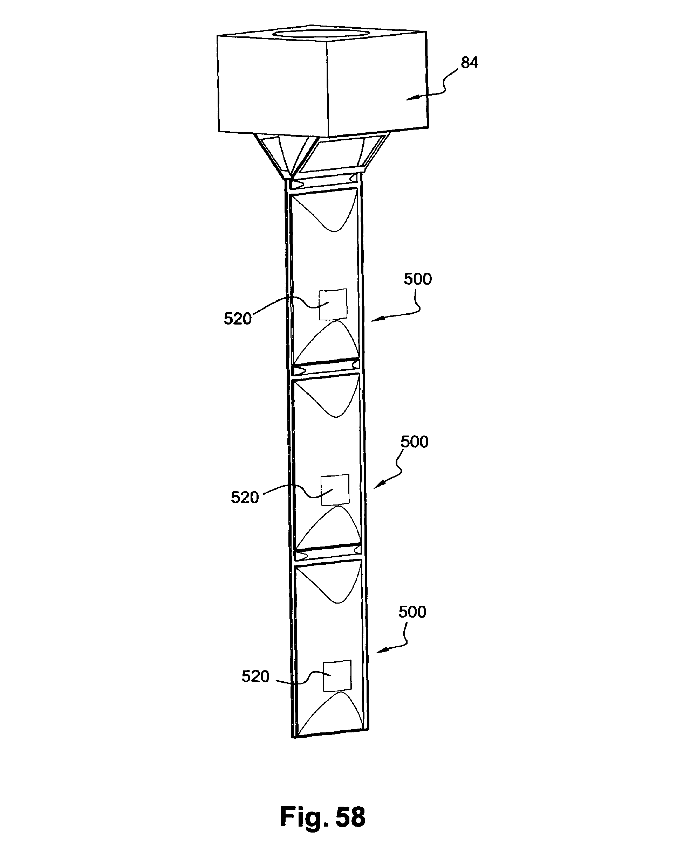

Figure 58 shows the sachets 500 resting in series vertically downward through the disposal shaft 84 which includes the means for packing in the sachets 500.

Each sachet bears a label 520 which is added to the labelling of each of the containers housed in the sachets. Given the small size of the labels 402 affixed to the containers, the additional labels 520 make it possible to add much of the information mentioned previously, such as the destination of the sachets.

The information on the labels 520 can be printed after the sachets have left by means of an associated printer outside the workstation, not shown in Figure 58.

### Waste recovery

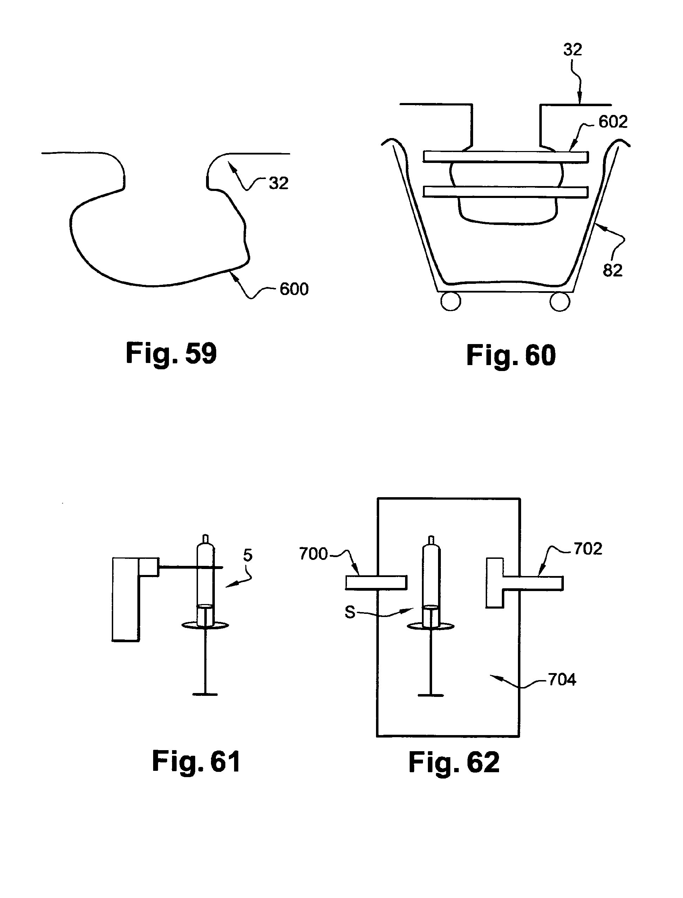

Figure 59 diagrammatically represents a bag 600 for recovering the variety of waste that leaves via the shaft 80. All this waste comprises contaminated elements that are removed through a standardized type F port or shaft, making it possible in particular to collect liquids. When the sachet is full, the operator implements a shaft locking mechanism and the bag is closed in a sealtight manner to then be dealt with.

Figure 60 represents a bin 82 with automatic sealtight closure of "Paxxo" type, combined with a type F shaft. Seals 602 are in particular provided.

### Verification and validation

Since the systems and the workstation are entirely automated, certain customers may ask for additional quality control tests to be carried out so as to be certain that the cytotoxic drug prepared at the dose for the patient is the correct drug and is delivered in the correct concentration.

This verification/validation can be performed inside the preparation area and, to this end, various techniques or technologies are available, such as Raman, infrared and ultraviolet spectrometry, HPLC analysis, analysis by colorimetry, and so on.

The verification can include a weight verification, for each reconstituted vial, of the final syringe, of the final IV bag. It includes the reading of the labels on the vials with a barcode scanner if necessary. Optionally coloured syringes make it possible to improve quality control.

The final validation of the product at the end can be performed without taking a sample directly from the final container, using the Raman technology, or by taking a sample and placing it in a special sampling vial using a kit for this purpose. When the validation is done by UV spectrometry, the vial or test cells have suitable optical properties.

When using Raman spectrometry, the laser light source is placed outside the station, as illustrated in Figure 61, and the liquid can be tested directly through the container of the final product. The test laser beam can be allowed to penetrate, by directing it directly onto the liquid inside the container (syringes or bags).

This can also be done by orienting the beam into a corresponding area of the adaptation pin.

It is even possible to have the light source 700 and the reception module 702 with the sample placed between these two elements, the whole being placed in a black chamber 704 during the analysis (Figure 62).

When the analysis requires a sample of the final product to be taken, the latter may be placed in a vial or test cell of very small dimensions which remains inside the workstation, the latter being equipped with means for handling and washing the test cell after the validation test so that the test cell can be reused.

When the validation means are of the UV and/or IR spectrometry type, and in order not to use a pipette to take the sample to be tested, the robot can, for example, inject 1 ml into a swabbable port which will be directly connected to the spectrometer. In order to avoid any contamination, a neutral liquid is injected between two tests in order to clean the pipes and tips.

## Claims

1. Automated workstation (10) for the preparation, starting from different substances, in powder and/or liquid form, each of which is contained in a vessel such as a vial (F) or a bag, of a final product for medical or pharmaceutical use of a determined composition and/or in determined quantities, the final product being packaged in a container such as an IV bag for intravenous IV use or a syringe (S), the workstation comprising at least:
- an isolated chamber (16) which delimits different internal functional areas;
- a storage area (20), for storing the filled vessels and containers;
- a transfer area (24);
- a preparation area (22), for preparing the final products, which communicates at least with said storage area (20) via said transfer area (24), and which comprises means for rapidly decontaminating the preparation area and its contents, said preparation area being isolated from the outside;
- a lock device (26) for unloading the final products from the workstation (10);
and at least one robot (R1, R2) or one programmable logic controller for handling the objects contained in the storage area (20) and/or in the preparation area (22),
**characterized in that** the workstation further comprises;
. - a loading area (18), in particular for the vessels and containers, comprising means for rapidly decontaminating the loading area (18) and its contents, said loading area (18) being isolated from the outside and communicating with the storage area (20);
- a lock device for introducing the vessels and containers into the loading area (18).

2. Automated workstation according to claim 1, **characterized in that** it comprises at least two robots (R1, R2), or programmable logic controllers for handling objects, one of which is arranged in the storage area (20) and the other one of which is arranged in the preparation area (22).

3. Automated workstation according to claim1, **characterized in that** said loading area (18) also communicates with said preparation area (22).

4. Automated workstation according to claim 1, **characterized in that** it comprises labelling means (30), arranged in the storage area, which comprise a printer, in particular for printing labels in situ designed to be affixed to the vessels and/or containers.

5. Automated workstation according to according to claim 1, **characterized in that** it comprises refrigerated storage means arranged in the storage area (20).

6. Automated workstation according to claim 1, **characterized in that** it comprises means for packaging the final products in a protective envelope (500), these packaging means being arranged in the final product preparation area.

7. Automated workstation according to claim 1, **characterized in that** it is housed in a flexible protective and isolating tent (40).

8. Automated workstation according to claim 1, **characterized in that**, for the preparation of the products, it contains closed needle-less transfer systems each of which mechanically prevents environmental pollutants from being transferred into the said system and hazardous substances or vapour concentrations from escaping towards the outside of the said system.

9. Automated workstation according to claim 1, **characterized in that** it comprises analysis, checking and validation means which make it possible for a final product, after it has been prepared and before it leaves the automated station (10), to verify the conformity of the said final product.

10. Automated workstation according to claim 9, **characterized in that** the checking and validation of the product is performed without taking a sample directly from the final container, using the Raman spectrometry technology.

11. Automated workstation according to claim 9, **characterized in that** said verification includes a weight verification.

12. Automated workstation according to claim 1, **characterized in that** two of said areas are separated by a rotating vertical carrousel (58) forming a rotating door with multiple shelving units which making it possible to transfer different elements and components from one area to the other area.

13. Automated workstation according to claim 12, **characterized in that** the carrousel (58) forming a rotating door has two opposing faces, each of the said opposite faces being fitted, with a preparation wheel (300) equipped with preparation means.

14. Automated workstation according to claim 1, **characterized in that** the introduction lock device is allows for the bio-decontamination of all the external or outside surfaces of all the elements and components that are placed inside the lock device by spraying suitable decontamination products.

## Patentansprüche

1. Automatisierte Arbeitsstation (10) für die Herstellung eines Endprodukts für den medizinischen oder pharmazeutischen Gebrauch mit einer festgelegten Zusammensetzung und/oder in festgelegten Mengen, die mit verschiedenen Substanzen in Pulverform und/oder flüssiger Form, wovon jede in einem Gefäß wie zum Beispiel einem Fläschchen (F) oder einem Beutel enthalten sind, beginnt, wobei das Endprodukt in einem Behälter wie zum Beispiel einem IV-Beutel für eine intravenöse Anwendung, IV-Anwendung, oder einer Spritze (S) verpackt ist, wobei die Arbeitsstation mindestens Folgendes umfasst:
- eine isolierte Kammer (16), die verschiedene innere Funktionsbereiche abgrenzt;
- einen Speicherbereich (20) zum Speichern der gefüllten Gefäße und Behälter;
- einen Übergabebereich (24);
- einen Herstellungsbereich (22) zum Herstellen des Endprodukts, der über den Übergabebereich (24) mindestens mit dem Speicherbereich (20) in Verbindung steht und der Mittel für das schnelle Dekontaminieren des Herstellungsbereichs und seiner Inhalte umfasst, wobei der Herstellungsbereich von der äußeren Umgebung isoliert ist;
- eine Sperrvorrichtung (26) zum Entladen des Endprodukts aus der Arbeitsstation (10);
und mindestens einen Roboter (R1, R2) oder einen programmierbaren logischen Regler zum Handhaben der Gegenstände, die in dem Speicherbereich (20) und/oder in dem Herstellungsbereich (22) enthalten sind,
**dadurch gekennzeichnet, dass** die Arbeitsstation ferner Folgendes umfasst:
- einen Ladebereich (18) insbesondere für die Gefäße und die Behälter, der Mittel für die schnelle Dekontamination des Ladebereichs (18) und seiner Inhalte umfasst, wobei der Ladebereich (18) von der äußeren Umgebung isoliert ist und mit dem Speicherbereich (20) in Verbindung steht;
- eine Sperrvorrichtung zum Einführen der Gefäße und der Behälter in den Ladebereich (18).

2. Automatisierte Arbeitsstation nach Anspruch 1, **dadurch gekennzeichnet, dass** sie mindestens zwei Roboter (R1, R2) oder programmierbare logische Regler zum Handhaben der Gegenstände umfasst, von denen der eine in dem Speicherbereich (20) angeordnet ist und von denen der andere in dem Herstellungsbereich (22) angeordnet ist.

3. Automatisierte Arbeitsstation nach Anspruch 1, **dadurch gekennzeichnet, dass** der Ladebereich (18) auch mit dem Herstellungsbereich (22) in Verbindung steht.

4. Automatisierte Arbeitsstation nach Anspruch 1, **dadurch gekennzeichnet, dass** sie Etikettierungsmittel (30), die in dem Speicherbereich angeordnet sind, umfasst, die einen Drucker umfassen, insbesondere zum Drucken der Etiketten vor Ort, die entworfen sind, um auf die Gefäße und/oder Behälter aufgeklebt zu werden.

5. Automatisierte Arbeitsstation nach Anspruch 1, **dadurch gekennzeichnet, dass** sie gekühlte Speichermittel umfasst, die in dem Speicherbereich (20) angeordnet sind.

6. Automatisierte Arbeitsstation nach Anspruch 1, **dadurch gekennzeichnet, dass** sie Mittel zum Verpacken der Endprodukte in einer schützenden Hülle (500) umfasst, wobei die Verpackungsmittel in dem Endproduktherstellungsbereich angeordnet sind.

7. Automatisierte Arbeitsstation nach Anspruch 1, **dadurch gekennzeichnet, dass** sie in einem biegsamen, schützenden und isolierenden Zelt (40) untergebracht ist.

8. Automatisierte Arbeitsstation nach Anspruch 1, **dadurch gekennzeichnet, dass** sie für die Herstellung der Produkte geschlossene nadellose Übergabesystem enthält, von denen jedes mechanisch verhindert, dass Umweltschadstoffe in das System überführt werden und gefährliche Substanzen oder Dampfkonzentrationen in die äußere Umgebung des Systems austreten.

9. Automatisierte Arbeitsstation nach Anspruch 1, **dadurch gekennzeichnet, dass** sie Analyse-, Überprüfungs- und Validierungsmittel umfasst, die es für ein Endprodukt, nachdem es hergestellt worden ist und bevor es die Arbeitsstation (10) verlässt, ermöglichen, die Konformität des Endprodukts zu verifizieren.

10. Automatisierte Arbeitsstation nach Anspruch 9, **dadurch gekennzeichnet, dass** das Überprüfen und Validieren des Produkts unter Verwendung der Technik der Raman-Spektrometrie durchgeführt wird, ohne eine Probe direkt aus dem Behälter des Endprodukts zu nehmen.

11. Automatisierte Arbeitsstation nach Anspruch 9, **dadurch gekennzeichnet, dass** die Verifizierung eine Verifizierung des Gewichts umfasst.

12. Automatisierte Arbeitsstation nach Anspruch 1, **dadurch gekennzeichnet, dass** zwei der Bereiche durch ein sich vertikal drehendes Karussell (58) getrennt sind, das eine Drehtür mit mehreren Regaleinheiten bildet, die ermöglicht, dass verschiedene Elemente und Komponenten von einem Bereich an einen anderen Bereich übergeben werden.

13. Automatisierte Arbeitsstation nach Anspruch 12, **dadurch gekennzeichnet, dass** das Karussell (58), das eine Drehtür bildet, zwei gegenüberliegende Seiten besitzt, wobei jede der gegenüberliegenden Seiten mit einem Herstellungsrad (300) ausgestattet ist, das mit Herstellungsmitteln ausgerüstet ist.

14. Automatisierte Arbeitsstation nach Anspruch 1, **dadurch gekennzeichnet, dass** die Einführungssperrvorrichtung die Biodekontamination aller externen oder äußeren Oberflächen aller Elemente und Komponenten, die innerhalb der Sperrvorrichtung angeordnet sind, durch Versprühen geeigneter Dekontaminationsprodukte ermöglicht.

## Revendications

1. Station de travail automatisée (10) pour la préparation, à partir de différentes substances, en poudre et/ou sous forme liquide, dont chacune est contenue dans un récipient tel qu'un flacon (F) ou un sac, d'un produit final à usage médical ou pharmaceutique de composition et/ou en quantité déterminées, le produit final étant conditionné dans un conteneur tel qu'un sac à usage intraveineux (IV) ou une seringue (S), la station de travail comportant au moins :
- une enceinte isolée (16) qui délimite différentes zones intérieures fonctionnelles ;
- une zone (20) de stockage des récipients et des conteneurs chargés ;
- une zone de transfert (24) ;
- une zone (22) de préparation des produits finaux qui communique au moins avec ladite zone de stockage (20) via ladite zone de transfert (24), et qui comporte des moyens de décontamination rapide de la zone de préparation et de ce qu'elle contient, ladite zone de préparation étant isolée de l'extérieur ;
- un sas de déchargement (26) des produits finaux hors de la station de travail (10);
et au moins un robot (R1, R2) ou un automate de manipulation des objets contenus dans la zone de stockage (20) et/ou dans la zone de préparation (22)
**caractérisé en ce que** la station de travail comporte en outre :
- une zone (18) de chargement, notamment des récipients et des conteneurs, comportant des moyens de décontamination rapide de la zone de chargement (18) et de ce qu'elle contient, ladite zone de chargement (18) étant isolée de l'extérieur et communiquant avec la zone de stockage (20) ;
- un sas d'introduction des récipients et des conteneurs dans la zone de chargement (18).

2. Station de travail automatisée selon la revendication 1 , **caractérisée en ce qu'**elle comporte au moins deux robots (R1, R2), ou automates de manipulation d'objets, dont l'un est agencé dans la zone de stockage (20) et dont l'autre est agencé dans la zone de préparation (22).

3. Station de travail automatisée selon la revendication 1, **caractérisée en ce que** ladite zone de chargement (18) communique aussi avec ladite zone de préparation (22).

4. Station de travail automatisée selon la revendication 1, **caractérisée en ce qu'**elle comporte des moyens (30) d'étiquetage, agencés dans la zone de stockage, qui comportent une imprimante, notamment pour l'impression d'étiquettes in situ destinées à être apposées sur des récipients et/ou des conteneurs.

5. Station de travail automatisée selon la revendication 1, **caractérisée en ce qu'**elle comporte des moyens de stockage frigorifiques agencés dans la zone de stockage (20).

6. Station de travail automatisée selon la revendication 1, **caractérisée en ce qu'**elle comporte des moyens d'emballage des produits finaux dans une enveloppe (500) de protection, ces moyens d'emballage étant agencés dans la zone de préparation des produits finaux.

7. Station de travail automatisée selon la revendication 1, **caractérisée en ce qu'**elle est logée dans une tente souple (40) de protection et d'isolement.

8. Station de travail automatisée selon la revendication 1, **caractérisée en ce que**, pour la préparation des produits, elle contient des systèmes clos de transfert sans aiguilles dont chacun empêche mécaniquement le transfert de polluants environnementaux dans le système et l'échappement de substances dangereuses ou de concentration de vapeur vers l'extérieur du système.

9. Station de travail automatisée selon la revendication 1, **caractérisée en ce qu'**elle comporte des moyens d'analyse, de vérification et de validation qui permettent pour un produit final, après sa préparation et avant sa sortie de la station automatisée (10), de vérifier la conformité de ce produit final.

10. Station de travail automatisée selon la revendication 9, **caractérisée en ce que** la validation finale du produit peut être effectuée sans prélèvement d'échantillon directement dans le conteneur final, au moyen de la technologie Raman.

11. Station de travail automatisée selon la revendication 9, **caractérisée en ce que** la vérification comporte une vérification de poids.

12. Station de travail automatisée selon la revendication 1, **caractérisée en ce que** deux desdites zones sont séparées par un carrousel vertical rotatif (58) à étagères multiples, formant porte tournante, permettant de transférer différents éléments et composants d'une zone vers l'autre zone.

13. Station de travail automatisée selon la revendication 12, **caractérisée en ce que** le carrousel vertical rotatif (58) formant porte tournante comporte deux faces opposées dont chacune est équipée d'une roue de préparation (300) munie de moyens de préparation,

14. Station de travail automatisée selon la revendication 1, **caractérisée en ce que** le sas d'introduction permet la biodécontamination de toutes les surfaces externes ou extérieures de tous les éléments et composants qui sont placés à l'intérieur du sas, par pulvérisation de produits adaptés de décontamination.
